(19) Europäisches Patentamt
European Patent Office
Office européen des brevets

(11) EP 3 886 859 B1

(12) # FASCICULE DE BREVET EUROPEEN

(45) Date de publication et mention de la délivrance du brevet:
**26.06.2024 Bulletin 2024/26**

(21) Numéro de dépôt: **19858717.2**

(22) Date de dépôt: **28.11.2019**

(51) Classification Internationale des Brevets (IPC):
***A61K 31/517*** *(2006.01)* ***A61P 39/02*** *(2006.01)*
***C07D 417/14*** *(2006.01)* ***A61K 9/00*** *(2006.01)*

(52) Classification Coopérative des Brevets (CPC):
**A61K 31/517; A61P 39/02; C07D 417/14;**
Y02A 50/30

(86) Numéro de dépôt international:
**PCT/EP2019/082976**

(87) Numéro de publication internationale:
**WO 2020/109510 (04.06.2020 Gazette 2020/23)**

(54) **NOUVELLES DIHYDROQUINAZOLINONES AYANT UNE ACTIVITE PROTECTRICE VIS-A-VIS DE TOXINES AU MODE D'ACTION INTRACELLULAIRE, DE VIRUS ET DE BACTERIES INTRACELLULAIRES**

NEUE DIHYDROCHINAZOLINONE MIT PROTEKTIVER WIRKUNG GEGEN INTRAZELLULÄR WIRKENDE ENDOTOXINE, INTRAZELLULÄRE VIREN UND BAKTERIEN

NEW DIHYDROQUINAZOLINONES EXHIBITING PROTECTIVE ACTIVITY AGAINST INTRACELLULAR-ACTING TOXINS, INTRACELLULAR VIRUSES AND BACTERIA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.11.2018 FR 1872016**

(43) Date de publication de la demande:
**06.10.2021 Bulletin 2021/40**

(73) Titulaire: **Commissariat à l'Energie Atomique et aux Energies Alternatives**
**75015 Paris (FR)**

(72) Inventeurs:
- **CINTRAT, Jean-Christophe**
  **91430 IGNY (FR)**
- **BARBIER, Julien**
  **91190 GIF-SUR-YVETTE (FR)**
- **GILLET, Daniel**
  **92160 ANTONY (FR)**
- **PRUVOST, Alain**
  **78460 CHOISEL (FR)**
- **COUHERT, Audrey**
  **38090 VILLEFONTAINE (FR)**
- **TEPSHI, Livia**
  **92160 ANTONY (FR)**
- **VINCK, Robin**
  **91191 GIF SUR YVETTE Cedex (FR)**

(74) Mandataire: **Gevers & Orès**
**Immeuble le Palatin 2**
**3 Cours du Triangle**
**CS 80165**
**92939 Paris La Défense Cedex (FR)**

(56) Documents cités:
**EP-A1- 2 722 047 EP-A1- 2 722 328**
**EP-A1- 3 085 374**

- **ROMAIN NOEL ET AL: "N -Methyldihydroquinazolinone Derivatives of Retro-2 with Enhanced Efficacy against Shiga Toxin", JOURNAL OF MEDICINAL CHEMISTRY, vol. 56, no. 8, 25 avril 2013 (2013-04-25) , pages 3404-3413, XP055095031, ISSN: 0022-2623, DOI: 10.1021/jm4002346**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- J.M. LORD ET AL: "Retrograde transport of toxins across the endoplasmic reticulum membrane", BIOCHEMICAL SOCIETY TRANSACTIONS, vol. 31, no. 6, 1 décembre 2003 (2003-12-01), pages 1260-1262, XP055621330, GB ISSN: 0300-5127, DOI: 10.1042/bst0311260 cité dans la demande
- NEETU GUPTA ET AL: "( S )- N -Methyldihydroquinazolinones are the Active Enantiomers of Retro-2 Derived Compounds against Toxins", ACS MEDICINAL CHEMISTRY LETTERS, 4 décembre 2013 (2013-12-04), XP055095054, ISSN: 1948-5875, DOI: 10.1021/ml400457j
- DESAI DHIMANT ET AL: "Inhibition of diverse opportunistic viruses by structurally optimized retrograde trafficking inhibitors", BIOORGANIC & MEDICINAL CHEMISTRY, vol. 27, no. 9, 13 March 2019 (2019-03-13) , pages 1795-1803, XP085652495, ISSN: 0968-0896, DOI: 10.1016/J.BMC.2019.03.026

## Description

La présente invention a pour objet une nouvelle famille de composés du type 2,3-dihydroquinazolin-4(1*H*)-one et leur utilisation en tant qu'inhibiteurs des effets toxiques des toxines à activité intracellulaire, comme par exemple la ricine, les toxines de Shiga et la toxine cholérique, utilisant le transport rétrograde pour intoxiquer les cellules, ou des virus ou bactéries utilisant le transport rétrograde et/ou dépendant de la syntaxine 5 pour infecter les cellules, notamment les virus ou bactéries entrant dans les cellules par endocytose, ou des parasites intracellulaires.

Les toxines à activité intracellulaire qui utilisent le transport rétrograde représentent un risque majeur de santé publique et sont associées pour certaines à plus d'un million de décès chaque année dans le monde. Ces toxines sont notamment la ricine (produite dans les graines de la plante *Ricinus communis*), la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile *(E. coli)*.

La ricine est une glycoprotéine de 66 kDa composée de deux chaînes polypeptidiques reliées par un pont disulfure **(Figure 1A).** La chaîne B (RTB, lectine de 262 résidus) permet à la toxine de se lier aux glycolipides et glycoprotéines des membranes cellulaires et d'assurer son entrée dans la cellule **(Figure 1A).** La chaîne A (RTA, 267 acides aminés) assure la fonction enzymatique N-glycosidase de la ricine, catalyse l'élimination de l'adénine 4324 de l'ARN 28S des cellules ciblées et provoque l'arrêt de la synthèse des protéines.

Les toxines de Shiga incluent la toxine de Shiga (Stx) produite par *Shigella dysenteriae* et les toxines Shiga-like 1 (Stx1) et 2 (Stx2) produites par des souches entérohémorragiques d'*E. coli.* Les toxines Stx et Stx1 sont à 99% identiques alors que les Stx1 et Stx2 partagent seulement 56% d'identité de leur séquence en acides aminés. La sous-unité A de la toxine (StxA) porte la même activité enzymatique que la ricine et cible de manière identique l'ARN 28S **(Figure 1B).** La sous-unité B (StxB), qui se présente sous forme pentamérique, permet la liaison de la toxine avec la cellule *via* son interaction avec le globo-triaosylcéramide Gb3, ce qui assure son internalisation et son routage intracellulaire.

Après liaison à ses récepteurs membranaires, la ricine est internalisée dans ces cellules par de multiples voies d'endocytose pour atteindre le réseau *trans*-golgien où elle est acheminée vers le réticulum endoplasmique (RE) par transport rétrograde (Johannes, L. et al., Cell 2008, 135, 1175-87). Les toxines de Shiga sont internalisées quant à elles par une voie d'endocytose unique et atteignent également l'appareil de Golgi puis le réticulum endoplasmique (*ibid.*).

Les toxines sont alors partiellement dépliées et la chaîne/sous-unité A est transloquée dans le cytosol (Lord, J.M. et al., Biochemical Society Transactions 2003, 31, 1260). L'étape ultime de l'action de ces toxines a donc lieu dans le cytoplasme des cellules, les toxines se fixent sur les ribosomes avec une grande efficacité et clivent l'adénine 4324 de l'ARN 28S de la sous-unité 60S du ribosome. Cette dépurination de l'ARN 28S provoque l'arrêt de la synthèse des protéines et conduit à la mort cellulaire.

Pour contrer la menace posée par ces toxines, plusieurs types d'antitoxines ont été développés : anticorps neutralisants, inhibiteurs de l'activité enzymatique (petites molécules, analogues de substrat), mimes solubles de récepteurs et composés chimiques agissant sur les cellules ciblées par la toxine.

Ces dernières années, la recherche de nouvelles molécules visant à bloquer le routage intracellulaire des toxines à activité intracellulaire s'est accélérée. Le principal avantage de ce type de molécules est leur activité de type large spectre puisque ces molécules peuvent protéger efficacement les cellules contre les différentes toxines qui utilisent la voie rétrograde.

Dans le cadre de ses recherches sur des composés bloquant le transport rétrograde et plus particulièrement d'études sur des composés plus avantageux que ceux des travaux précités, la Demanderesse a identifié une nouvelle famille de composés dérivés de 2,3-dihydroquinazolin-4(1*H*)-one, portant un phényle spécifiquement substitué en position ortho ou méta, qui présentent de manière inattendue une activité biologique supérieure à celle de Retro-2.1, la plus active des molécules déjà décrites (ROMAIN NOEL ET AL: "N -Methyldihydroquinazolinone Dérivatives of Retro-2 with Enhanced Efficacy against Shiga Toxin",JOURNAL OF MEDICINAL CHEMISTRY, vol. 56, no. 8, 25 avril 2013, pages 3404-3413; NEETU GUPTA ET AL: "( S )- N -Methyldihydroquinazolinones are the Active Enantiomers of Retro-2 Derived Compounds against Toxins",ACS MEDICINAL CHEMISTRY LETTERS, 4 décembre 2013,ISSN: 1948-5875, DOI: 10.1021/ml400457j), et donc un intérêt marqué pour la prévention et/ou le traitement des intoxications à au moins une toxine à mode d'action intracellulaire utilisant le transport rétrograde pour infecter les cellules eucaryotes de mammifères, mais également pour la prévention et/ou le traitement des infections par des virus ou bactéries utilisant le transport rétrograde et/ou dépendant de la syntaxine 5 pour infecter les cellules, notamment les virus ou bactéries entrant dans les cellules par endocytose, ou à des parasites intracellulaires.

Ainsi, la présente invention se rapporte à un composé de formule générale (I) :

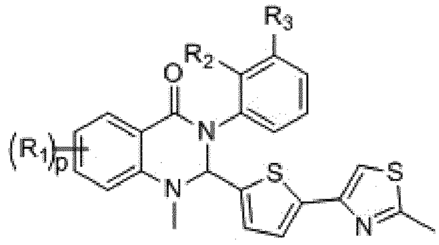

(I)

où :

- p est égal à 1, 2 ou 3 ;
- $R_1$ représente à chaque occurrence, de façon indépendante, un atome d'hydrogène, un atome d'halogène, un radical alcoxy de 1 à 3 atomes de carbone, notamment un groupe méthoxy, $-NO_2$, ou $-NH_2$ ;
- $R_2$ et $R_3$ représentent indépendamment l'un de l'autre un groupe choisi parmi H, -OH, $-OR_4$, $-NH_2$, $-NHR_5$, $-SO_2-NH_2$, $-SO_2-NH-R_6$, à la condition que l'un au moins de $R_2$ et $R_3$ ne représente pas H ;
- $R_4$, $R_5$ et $R_6$ représentent indépendamment les uns des autres un groupe de formule (II) $-L-(X)_i-(PEG)-(Y)_j-Z$, où :
  - i et j représentent indépendamment l'un de l'autre 0 ou 1 ;
  - L représente -C(=O)- ou $-C(=O)-(CH_2)_k-C(=O)-$, avec k étant égal à 1, 2 ou 3, en particulier 2 ;
  - X et Y représentent indépendamment l'un de l'autre un poly(acide lactique) ou un poly(acide lactique-co-acide glycolique) ;
  - PEG représente un poly(éthylène glycol) ;
  - Z représente un groupe choisi parmi H, un alkyle en $C_1$ à $C_3$, -OH, un O-alkyle en $C_1$ à $C_3$, ou $-L-R_e$, où L est défini tel que précédemment et $R_e$ est un résidu de formule (I) relié audit groupe $-L-(X)_i-(PEG)-(Y)_j-$ défini précédemment par l'intermédiaire de son groupe $R_2$ ou $R_3$, ledit groupe $R_2$ ou $R_3$ étant -OH, $-NH_2$ ou - $SO_2-NH_2$;

ainsi que les formes stéréoisomères, les mélanges de formes stéréoisomères ou leurs sels pharmaceutiquement acceptables.

La présente invention se rapporte également à un composé de formule générale (I) :

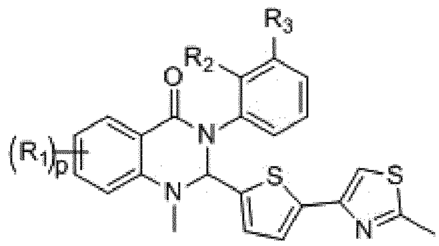

(I)

où :

- p est égal à 1, 2 ou 3 ;
- $R_1$ représente à chaque occurrence, de façon indépendante, un atome d'hydrogène, un atome d'halogène ou un radical alcoxy de 1 à 3 atomes de carbone, notamment un groupe méthoxy ;
- $R_2$ et $R_3$ représentent indépendamment l'un de l'autre un groupe choisi parmi H, -OH, $-NH_2$, $-SO_2-NH_2$, à la condition que l'un au moins de $R_2$ et $R_3$ ne représente pas H;

ainsi que les formes stéréoisomères, les mélanges de formes stéréoisomères ou leurs sels pharmaceutiquement acceptables.

Selon un mode de réalisation, $R_2$ représente un groupe choisi parmi -OH, $-NH_2$, - $SO_2-NH_2$, et $R_3$ représente un groupe choisi parmi H, -OH, $-NH_2$, $-SO_2-NH_2$.

Selon un mode de réalisation, $R_3$ représente un atome d'hydrogène. Le composé de formule générale (I) selon l'invention est alors de formule suivante :

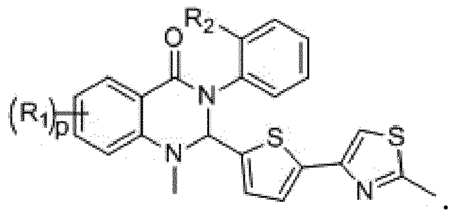

Selon un mode de réalisation, $R_2$ représente un atome d'hydrogène. Le composé de formule générale (I) selon l'invention est alors de formule suivante :

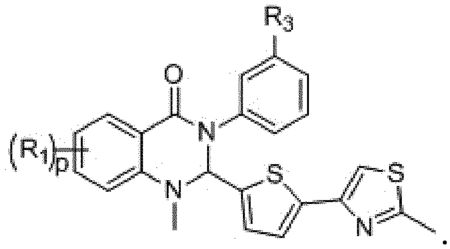

Selon un mode de réalisation, $R_2$ et $R_3$ représentent indépendamment l'un de l'autre un groupe choisi parmi -OH, $-NH_2$, $-SO_2-NH_2$, $R_2$ et $R_3$ représentant notamment -OH.
Selon un mode de réalisation, p est égal à 1.
Selon un mode de réalisation, le composé de formule générale (I) selon l'invention est de formule suivante :

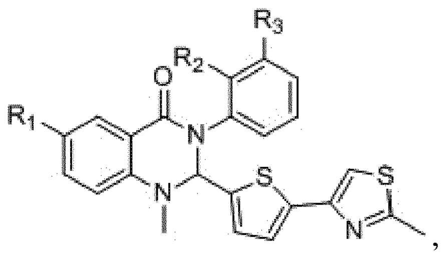

$R_1$, $R_2$ et $R_3$ étant tels que définis précédemment, $R_2$ et $R_3$ représentant notamment, indépendamment l'un de l'autre, un groupe choisi parmi -OH, $-NH_2$, $-SO_2-NH_2$, $R_2$ et $R_3$ représentant par exemple -OH.
Selon un mode de réalisation particulier, le composé de formule générale (I) selon l'invention est de formule suivante :

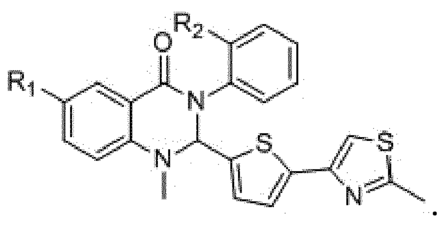

Selon un mode de réalisation particulier, le composé de formule générale (I) selon l'invention est de formule suivante :

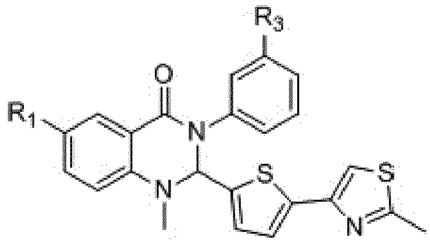
.

Selon un mode de réalisation, $R_1$ représente un atome d'halogène, en particulier un atome de fluor.

Selon un mode de réalisation, le composé de formule générale (I) selon l'invention est de formule (Ia) suivante :

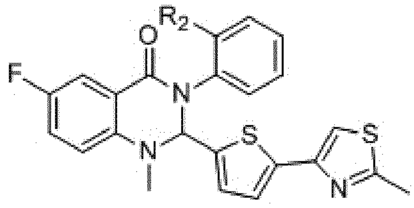
(Ia).

Selon un mode de réalisation, le composé de formule générale (I) selon l'invention est de formule (Ib) suivante :

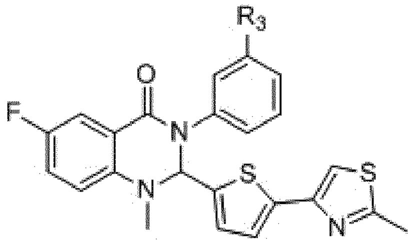
(Ib).

Selon un mode de réalisation, le composé de formule générale (I) selon l'invention est choisi parmi :

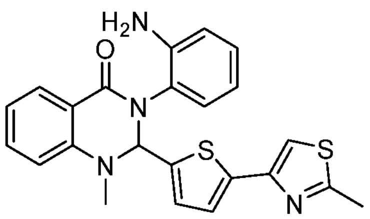
4

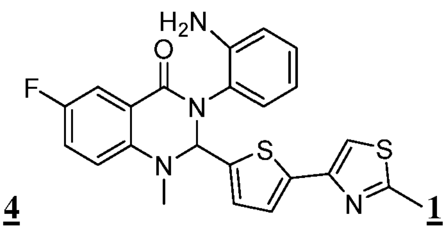
1

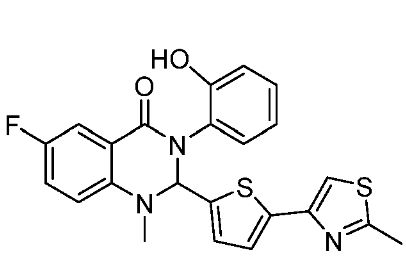
2

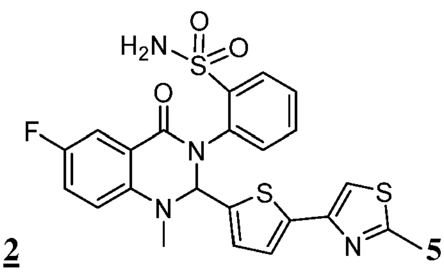
5

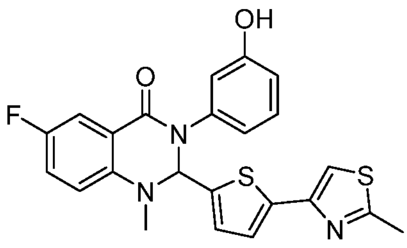 3 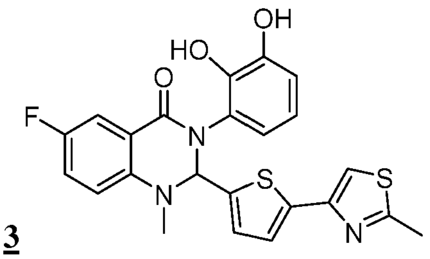 7.

Selon un mode de réalisation, l'invention concerne un composé de formule générale (I) dans laquelle $R_2$ et $R_3$ représentent indépendamment l'un de l'autre un groupe choisi parmi H, -OH, $-OR_4$, $-NH_2$, $-NHR_5$, $-SO_2-NH_2$, $-SO_2-NH-R_6$, à la condition que l'un au moins de $R_2$ et $R_3$, en particulier $R_2$, représente un groupe choisi parmi$-OR_4$, $-NHR_5$ et $-SO_2-NH-R_6$, $R_4$, $R_5$ et $R_6$ étant définis tel que précédemment.

En ce qui concerne le groupe de formule (II) $-L-(X)_i-(PEG)-(Y)_j-Z$, L représente notamment -C(=O)- lorsque $R_2$ ou $R_3$ représente $-NHR_5$ ou $-SO_2-NH-R_6$, et L représente notamment$-C(=O)-(CH_2)_k-C(=O)-$ lorsque $R_2$ ou $R_3$ représente $-OR_4$.

Selon un mode de réalisation, le groupe de formule (II) $-L-(X)_i-(PEG)-(Y)_j-Z$ est choisi parmi -L-PEG, -L-PEG-PLA, et -L-PLGA-PEG-PLGA, avec L représentant notamment -C(=O)- lorsque $R_2$ ou $R_3$ représente $-NHR_5$ ou $-SO_2-NH-R_6$, ou notamment$-C(=O)-(CH_2)_k-C(=O)-$ lorsque $R_2$ ou $R_3$ représente $-OR_4$.

Selon un mode de réalisation, le groupe de formule (II) $-L-(X)_i-(PEG)-(Y)_j-Z$ est choisi parmi $-C(=O)-(CH_2-CH_2-O)_m-CH_3$, notamment lorsque $R_2$ ou $R_3$ représente $-NHR_5$ ou $-SO_2-NH-R_6$, et $-C(=O)-(CH_2)_k-C(=O)-(O-CH_2-CH_2)_m-OCH_3$, notamment lorsque $R_2$ ou $R_3$ représente $-OR_4$, m étant compris de 1 à 500, notamment de 4 à 500, m étant en particulier compris de 30 à 60, plus particulièrement de 45.

Selon un mode de réalisation, le composé de formule générale (I) est de formule (III) suivante :

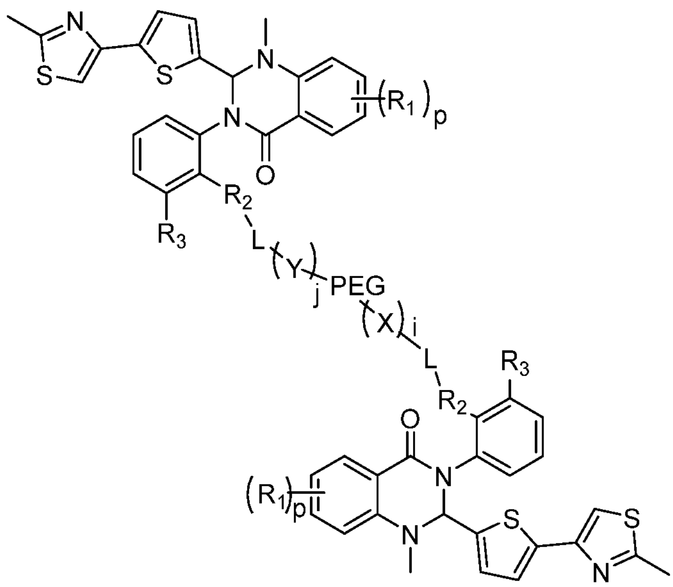 (III),

les groupes mentionnés dans la formule (III) étant tels que définis plus haut.

Selon un mode de réalisation, le composé de formule générale (I) selon l'invention est choisi parmi :

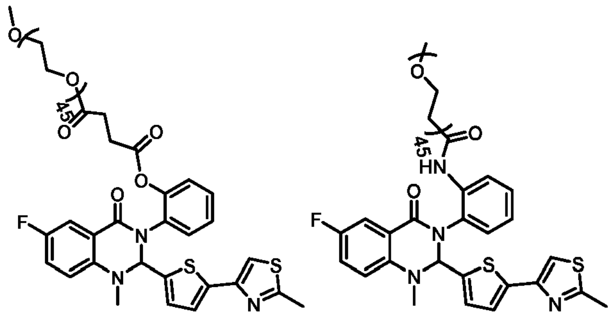

R = $PEG_{450}$
R = PEG-PLA
R = PLGA-PEG-PLGA
en particulier $PLGA_{1036}$-$PEG_{1450}$-$PLGA_{1036}$

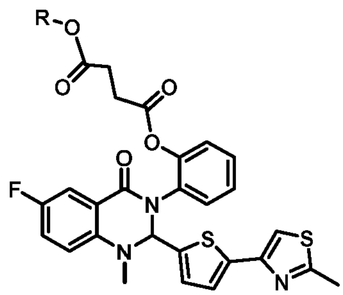

et (Composé **2**)-PLGA1036-PEG1450-PLGA1036-(Composé **2**) ou (Composé **2**)-C(=O)-$(CH_2)_2$-C(=O)-PLGA1036-PEG1450-PLGA1036-C(=O)-$(CH_2)_2$-C(=O)-(Composé 2), en particulier de formule suivante :

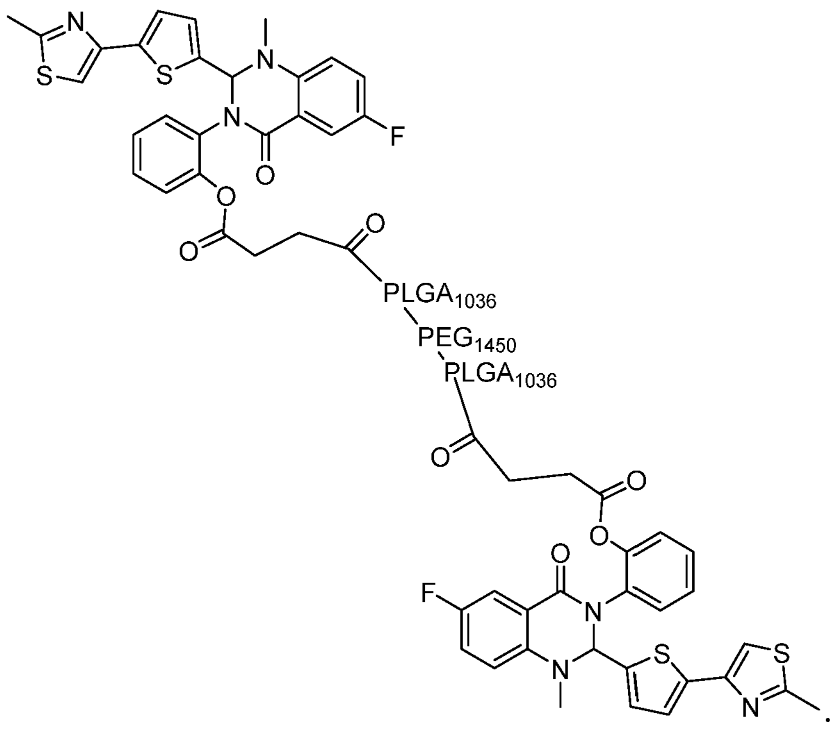

Selon un autre aspect, l'invention concerne une composition pharmaceutique ou médicament comprenant au moins un composé de formule générale (I) tel que défini précédemment, en tant que principe actif, et un véhicule pharmaceutiquement acceptable, ladite composition pharmaceutique ou ledit médicament étant notamment adapté à une administration par les voies aérienne, orale, parentérale ou locale.
Il est à noter que tous les modes de réalisation mentionnés ci-dessus à propos du composé de formule générale (I) s'appliquent également ici, seuls ou en combinaison.
Selon un autre aspect, l'invention concerne un composé de formule générale (I) tel que défini précédemment, pour son utilisation pour la prévention et/ou le traitement des désordres induits par les toxines à mode d'action intracellulaire utilisant le transport rétrograde, ou par les virus ou bactéries utilisant le transport rétrograde et/ou dépendant de la syntaxine 5 pour infecter les cellules, notamment les virus ou bactéries entrant dans les cellules par endocytose, ou par des parasites intracellulaires.

Il est à noter que tous les modes de réalisation mentionnés ci-dessus à propos du composé de formule générale (I) s'appliquent également ici, seuls ou en combinaison.

Selon un mode de réalisation, l'invention concerne un composé de formule générale (I) tel que défini précédemment, pour son utilisation pour la prévention et/ou le traitement des désordres induits par les toxines à mode d'action intracellulaire utilisant le transport rétrograde.

Selon un mode de réalisation particulier, lesdites toxines à mode d'action intracellulaire utilisant le transport rétrograde sont choisies parmi la ricine, la toxine de Shiga et les toxines Shiga-like (Stxs) produites par *Shigella dysenteriae* (Stx) et *E. coli* (Stx1 et Stx2, Stx, Stx1a, Stx2a, Stx2c, Stx2d, Stx2e tels que décrites par exemple par Melton-Celsa, *Microbiol Spectr.* 2014 ; 2(2)), la toxine cholérique (Ctx de *Vibrio cholerae* responsable du choléra), la toxine pertussique (*Bordetella pertussis* agent de la coqueluche), la cytotoxine subtilase et l'entérotoxine thermolabile (*E. coli*).

Selon un mode de réalisation, l'invention concerne un composé de formule générale (I) tel que défini précédemment, pour son utilisation pour la prévention et/ou le traitement des désordres induits par les virus ou bactéries utilisant le transport rétrograde et/ou dépendant de la syntaxine 5 pour infecter les cellules, notamment les virus ou bactéries entrant dans les cellules par endocytose, ou les parasites intracellulaires.

Selon un mode de réalisation particulier, les virus sont les virus pox, notamment le virus de variole et le virus Vaccinia, les cytomégalovirus, les adénovirus, notamment les virus adéno-associés, en particulier de sérotype 2, les polyomavirus, notamment le polyomavirus JC et le polyomavirus BK, les papillomavirus, les filovirus, notamment les virus Ebola et le virus Marburg, les entérovirus, notamment l'entérovirus 71, les virus de l'herpès, notamment le virus Herpes simplex de type 2, les virus du genre *Arenavirus,* notamment le virus de la chorioméningite lymphocytaire, les virus de la grippe, notamment les influenzavirus A.

Selon un mode de réalisation particulier, les virus sont les virus pox, notamment le virus de variole, le virus monkeypox, le virus Vaccinia et les leporipoxvirus, en particulier le virus de la myxomatose, les cytomégalovirus, les virus adéno-associés, en particulier de sérotype 2, les polyomavirus, notamment le polyomavirus JC et le polyomavirus BK, les papillomavirus, les filovirus, notamment les virus Ebola et le virus Marburg, les entérovirus, notamment l'entérovirus 71, les virus de l'herpès, notamment le virus Herpes simplex de type 2, les virus du genre *Arenavirus,* notamment le virus de la chorioméningite lymphocytaire, les virus de la grippe, notamment les influenzavirus A, les pneumovirus, notamment le virus respiratoire syncytial.

Selon un mode de réalisation particulier, les bactéries sont des bactéries de l'ordre des Chlamydiales, en particulier du genre Chlamydia, telles que *Chlamydia trachomatis, Chlamydophila pneumoniae, Chlamydophila psyttaci,* ou *Symkania,* comme *Symkania negevensis.*

Selon un mode de réalisation particulier, les désordres induits par les parasites intracellulaires sont la leishmaniose, notamment induite par des trypanosomes du genre *Leishmania,* en particulier *Leishmania infantum, Leishmania donovani* ou *Leishmania infantum/donovani hybrid.*

La présente invention se rapporte également à une méthode de prévention et/ou de traitement des désordres induits par les toxines à activité intracellulaire utilisant le transport rétrograde et comprenant l'administration d'une quantité efficace d'au moins un composé de formule générale (I) tel que défini précédemment.

Il est à noter que tous les modes de réalisation mentionnés ci-dessus à propos du composé de formule générale (I) s'appliquent également ici, seuls ou en combinaison.

***Synthèse***

Les composés de la présente invention peuvent être préparés selon des méthodes bien connues de l'homme du métier, y compris, mais sans s'y limiter, celles qui sont décrites ci-dessous, ou par des modifications de ces méthodes en appliquant des techniques standard connues de l'homme du métier de la synthèse organique. Les modifications et les substitutions appropriées seront bien connues, facilement apparentes, ou facilement accessibles de la littérature scientifique à l'homme du métier. En particulier, on peut trouver de telles méthodes dans R.C. Larock, Comprehensive Organic Transformations, Wiley-VCH Publishers, 1999.

Tous les procédés divulgués dans le cadre de la présente invention sont envisagés pour être pratiqués à n'importe quelle échelle, y compris le milligramme, le gramme, le multigramme, le kilogramme, le multikilogramme ou l'échelle industrielle commerciale.

Il est à noter que les composés de la présente invention peuvent comprendre un atome de carbone asymétriquement substitué, et peuvent être isolés sous des formes optiquement actives ou racémiques. Ainsi, toutes les formes chirales, diastéréoisomères, racémiques, isomères d'une structure sont envisagées, à moins que la stéréochimie ou la forme isomère spécifique ne soit spécifiquement indiquée. Il est bien connu de préparer et d'isoler de telles formes optiquement actives. Par exemple, les mélanges de stéréoisomères peuvent être séparés par des techniques standard,

y compris, mais sans s'y limiter, la résolution des formes racémiques, la chromatographie classique, en phase inverse et chirale, la formation préférentielle de sel, la recristallisation et autres, ou par synthèse chirale à partir de matières premières chirales ou par synthèse délibérée des centres chiraux cibles.

Les composés de la présente invention peuvent être préparés par diverses voies synthétiques.

Dans les réactions décrites ci-après, il peut être nécessaire de protéger les groupes fonctionnels réactifs, par exemple les groupes hydroxy, amino ou thio, lorsque ceux-ci sont présents dans le produit final, afin d'éviter leur participation à des réactions secondaires, non désirées. Les groupes de protection conventionnels peuvent être utilisés conformément à la pratique courante, par exemple, voir T.W. Greene and P. G. M. Wuts in Protective Groups in Organic Chemistry, 3rd ed., John Wiley and Sons, 1999; J. F. W. McOmie in Protective Groups in Organic Chemistry, Plénum Press, 1973.

Les réactifs et les composés de départ sont disponibles sur le marché, ou facilement synthétisés par des techniques bien connues de l'homme du métier. Tous les substituants, sauf indication contraire, sont tels que définis précédemment.

Les composés de formule générale (I) peuvent être obtenus selon la réaction suivante :

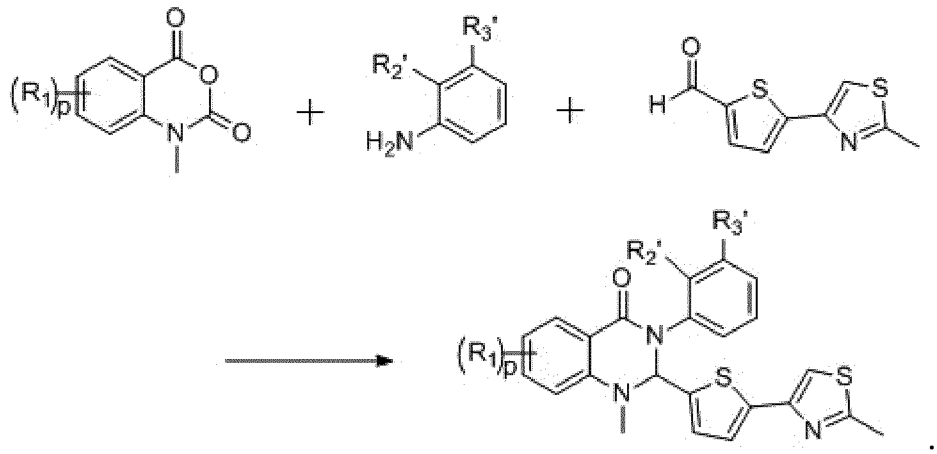

Cette réaction peut notamment être réalisée dans un solvant organique.

Selon un mode de réalisation, les trois composés de départ sont mélangés dans un solvant organique, notamment l'acide acétique.

Selon un mode de réalisation, le solvant organique est chauffé, notamment au reflux ou sous irradiations microondes, par exemple à une température comprise de 100°C à 180°C, en particulier de 110°C à 140°C, plus particulièrement de 120°C à 130°C.

La durée du chauffage est comprise de 10 minutes à 6 heures, notamment de 30 minutes à 5 heures, en particulier de 1 heure à 4 heures, plus particulièrement de 2 à 3 heures.

$R_1$ et p sont tels que définis plus haut.

$R_2$' et $R_3$' correspondent respectivement aux groupes $R_2$ et $R_3$, ou à des groupes de protections *ad hoc.*

Selon un mode de réalisation, $R_2$' et $R_3$' représentent indépendamment l'un de l'autre un groupe choisi parmi H, -OH, $-NO_2$, $-SO_2-NH_2$, à la condition que l'un au moins de $R_2$ et $R_3$ ne représente pas H.

Selon un mode de réalisation particulier, lorsque $R_2$' et/ou $R_3$' représentent $-NO_2$, $R_2$' et/ou $R_3$' sont alors converti en $R_2$ et/ou $R_3$ = $-NH_2$, notamment par l'une des techniques bien connues de l'homme du métier, par exemple par action de zinc au contact d'acide acétique.

***Définitions***

Tel qu'on l'utilise dans la présente description, le terme « environ » se réfère à un intervalle de valeurs de ± 10 % d'une valeur spécifique. A titre d'exemple, l'expression « environ 120 mg » comprend les valeurs de 120 mg ± 10 %, soit les valeurs de 108 mg à 132 mg.

Au sens de la présente description, les pourcentages se réfèrent à des pourcentages en poids par rapport au poids total de la formulation, sauf indication contraire.

Tel qu'on l'entend ici, les plages de valeur sous forme de « x-y » ou « de x à y » ou « entre x et y » incluent les bornes x et y ainsi que les entiers compris entre ces bornes. A titre d'exemple, « 1-5 », ou « de 1 à 5 » ou « entre 1 et 5 » désignent les entiers 1, 2, 3, 4 et 5. Les modes de réalisations préférés incluent chaque entier pris individuellement dans la plage de valeur, ainsi que toute sous-combinaison de ces entiers. A titre d'exemple, les valeurs préférées pour

« 1-5 » peuvent comprendre les entiers 1, 2, 3, 4, 5, 1-2, 1-3, 1-4, 1-5, 2-3, 2-4, 2-5, etc.

Tel qu'il est utilisé ici, le terme «sel pharmaceutiquement acceptable» se réfère à des sels qui sont, dans la portée d'un jugement médical sensé, appropriés pour le contact avec les tissus d'êtres humains et d'animaux sans toxicité excessive, irritation, réponse allergique, ou autres complications problématiques en proportion avec un rapport bénéfice / risque raisonnable.

Par sel pharmaceutiquement acceptable des composés de formule générale (I), on entend notamment les chlorhydrates, bromhydrates, sulfates ou bisulfates, phosphates ou hydrogénophosphates, acétates, oxalates, benzoates, succinates, fumarates, maléates, lactates, citrates, tartrates, gluconates, méthanesulphonates, benzène-sulphonates et paratoluène- sulphonates.

Par atome d'halogène, on entend les éléments chimiques du groupe VII du tableau périodique des éléments, notamment, le fluor, le chlore, le brome et l'iode.

Le terme radical alkyle de 1 à 3 ou 4 atomes de carbone désigne un radical hydrogénocarboné, linéaire ou ramifié ; on peut citer par exemple le méthyle, l'éthyle, le propyle, l'isopropyle ou le tertiobutyle.

Par « PEG » ou « poly(éthylène glycol) » ou « polyéthylène glycol », on entend notamment des groupes $-(CH_2-CH_2-O)_m-$, éventuellement terminés par un groupe choisi parmi H, un alkyle en $C_1$ à $C_3$, ou des groupes $-(-O-CH_2-CH_2)_m-$, éventuellement terminés par un groupe choisi parmi H, -OH, et un O-alkyle en $C_1$ à $C_3$, m étant choisi de 1 à 500, en particulier de 4 à 500, notamment de 30 à 60.

La masse molaire moyenne du PEG peut notamment être indiquée après le terme « PEG » après le nom, par exemple PEG-1450 (1 450 $g \cdot mol^{-1}$).

Par « PLA » ou « poly(acide lactique) » ou « acide polylactique », on entend notamment des groupes $-(C(=O)-CH(CH_3)-O)_p-$, éventuellement terminés par un groupe choisi parmi H, un alkyle en $C_1$ à $C_3$, ou des groupes $-(O-CH(CH_3)-C(=O))_p-$, éventuellement terminés par un groupe choisi parmi -OH, et un O-alkyle en $C_1$ à $C_3$, p étant choisi de 1 à 2000, notamment de 5 à 500, particulièrement de 10 à 50.

La masse molaire moyenne du PLA peut notamment être indiquée après le terme « PLA » après le nom, par exemple PLA-1036 (1 036 $g.mol^{-1}$).

Par « PLGA » ou « PLG », on entend un poly(acide lactique-co-acide glycolique), et notamment des groupes $-((C(=O)-CH(CH_3)-O)_p-(C(=O)-CH_2-O)_q)_r-$, éventuellement terminés par un groupe choisi parmi H, un alkyle en $C_1$ à $C_3$, ou des groupes $-((O-CH_2-C(=O))_q-(O-CH(CH_3)-C(=O))_p)_r-$, éventuellement terminés par un groupe choisi parmi - OH, et un O-alkyle en $C_1$ à $C_3$, p, q et r étant tels que le polymère PLGA soit statistique ou à blocs, en particulier statistique, et notamment tels que la masse molaire moyenne du PLGA, par exemple indiquée après le terme « PLGA » après le nom, soit comprise de 200 à 4000, notamment de 800 à 1200 $g.mol^{-1}$. p, q et r sont notamment tels que le pourcentage molaire d'acide lactique dans le PLGA est d'environ 20%. En particulier, p, q et r sont tels que le PLGA soit constitué de 12 unités d'acide lactique et de 3 unités d'acide glycolique.

FIGURES

La **Figure 1** représente les structures cristallographiques de la ricine (A, dont le nom dans la base de données des structures cristallographiques est pdb 2AAI) et de la Stx2 (B, pdb 1R4P).

La **Figure 2** est une représentation schématique du test cellulaire mis en oeuvre dans la partie expérimentale.

La **Figure 3** illustre la méthode de calcul de l'$IC_{50}$ réalisée dans la partie II des exemples ; les courbes de toxicité sont réalisées en l'absence d'inhibiteur (Contrôle) puis en présence d'inhibiteur à différentes concentrations.

## EXEMPLES

### I. Synthèse de composés selon l'invention

Tous les produits chimiques et solvants utilisés dans les synthèses sont de grade réactif et ont été utilisés sans purification supplémentaire. Le $CH_2Cl_2$ a été distillé sur hydrure de calcium avant utilisation. La verrerie a été séchée à la flamme sous vide et refroidie sous azote à température ambiante. Toutes les réactions ont été effectuées sous azote sec et contrôlées par TLC.

La purification a notamment été réalisée sur un CombiFlash avec un détecteur UV-vis et des colonnes RediSep. Les échantillons ont notamment été adsorbés sur de la Célite ou de la silice et chargés dans des cartouches à charge solide. Un gradient acétate d'éthyle/cyclohexane ou méthanol/dichlorométhane a notamment été utilisé. Les fractions ont notamment été collectées sur la base d'une détection à 254 nm.

L'analyse et la purification par CLHP-SM ont notamment été effectuées à l'aide d'un système Waters (module de gradient binaire 2525, dégazeur en ligne, gestionnaire d'échantillons 2767, détecteur à barrettes de photodiodes

2996) avec un système binaire pour le gradient de solvant. L'éluant était en particulier un gradient de (99,9 % eau / 0,1 % HCOOH) et (99,9 % MeCN / 0,1 % HCOOH) ou (99,9 % eau / 0,1 % HCOOH) et (99,9 % MeOH / 0,1 % HCOOH). Chaque composé a été déposé sur une colonne Zorbax SB-C18 de 100-4.6mm (5mm) équilibrée avec $H_2O$/MeCN ou $H_2O$/MeOH 95:5.

Ce système a été par exemple couplé à un système Waters Micromass ZQ avec un analyseur quadripolaire ZQ2000. L'ionisation a été réalisée par électropulvérisation et les autres paramètres étaient les suivants : température de la source 120 °C, tension du cône 20 V, et injection continue de l'échantillon à un débit de 0,3 mL par min. Les spectres de masse ont été enregistrés en mode ion positif et négatif dans la plage m/z 100-2000 et traités avec le logiciel Mass Lynx 4.0.

Les spectres infrarouges ont notamment été enregistrés sur un Spectrum Two équipé d'un UATR Two (Perkin Elmer), Diamond/ZnSe (1 réflexion).

Les analyses RMN ont notamment été réalisées sur un spectromètre Bruker Avance 400 Ultrashield. Les spectres 1H-NMR et 13C ont été enregistrés à température ambiante à 400 MHz et 100 MHz respectivement, les échantillons ont été dissous dans du DMSO-d6 ou $CDCl_3$ à une concentration d'environ 5 mM. Le signal singulet DMSO a été réglé à 2,50 ppm. Les déplacements chimiques sont donnés en ppm et les constantes de couplage en Hz. Les données spectrales sont cohérentes avec les structures associées.

**Préparation du composé 1**

**Synthèse du précurseur nitro 1'**

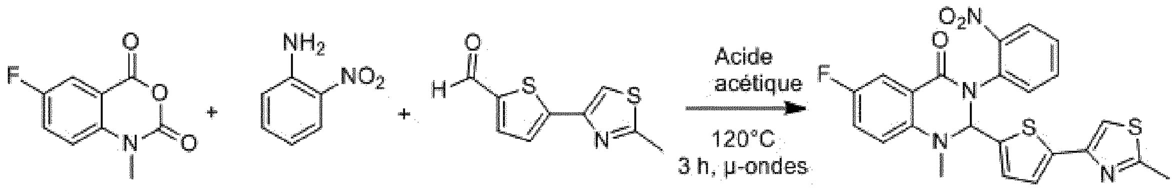


| Réactifs | éq. | M (g/mol) | d | n (mmol) | m (mg) | V (mL) |
|---|---|---|---|---|---|---|
| Anhydride 5-fluoro-N-méthylisatoïque | 1 | 195.15 | / | 1 | 195 | / |
| 2-nitroaniline | 1 | 138.13 | / | 1 | 138 | / |
| 5-(2-méthyl-1,3-thiazol-4-yl)-2-thiophènecarbaldéhyde | 1 | 209.00 | / | 1 | 209 | / |
| Acide acétique | / | / | / | / | / | 2 |

Le mélange a été agité à 120°C pendant 3 heures sous irradiation micro-ondes. Le résidu a été dissous dans de l'EtOAc et lavé successivement avec une solution saturée de $NaHCO_3$ (trois fois), de la saumure (deux fois) et de l'eau (une fois). La phase organique a été séchée sur $MgSO_4$, filtrée et concentrée. Le résidu a été purifié par chromatographie (dépôt solide), éluée par un mélange cyclohexane/EtOAc : 9/1 -> 1/1 (rendement : 62%).

**Synthèse du composé 1**

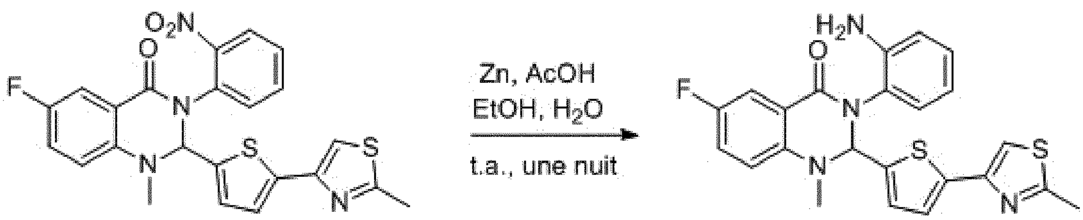

| Réactifs | éq. | M (g/mol) | n (mmol) | m (mg) | V (mL) |
|---|---|---|---|---|---|
| 1' | 1 | 480.07 | 0.44 | 273 | / |
| Zn | 30 | 65.39 | 13.2 | 860 | / |
| EtOH | / | / | / | / | 10 |
| $H_2O$ | / | / | / | / | 1.8 |
| AcOH | / | / | / | / | 0.9 |

Le mélange a été agité une nuit à température ambiante, à l'abri de la lumière.

Le résidu obtenu a été purifié par chromatographie (dépôt solide), éluée par un mélange cyclohexane/EtOAc : 95/5 -> 0/1, puis reprécipité par dissolution dans le minimum d'EtOAc et ajout de pentane (rendement : 63 %).
RMN $^1$H ($CDCl_3$) : 2,69 (s, 3H) ; 2,91 (s, 3H) ; 4 (s large, 2H) ; 5,76 (s, 1H) ; 6,54 (dd, 1H, J: 4, J: 8,9) ; 6,60 (td, 1H, J: 1.3, J: 7,8) ; 6,66 (d, 1H, J: 3,7) ; 6,75 (dd, 1H, J: 1.2, J: 8) ; 6,84 (dd, 1H, J: 1.4, J: 7,9) ; 7-7.1 (m, 3H) ; 7,1 (dd, 1H, J: 3.1, J: 8,7) ; 7.72 (dd, 1H, J: 3, J: 8,6).
RMN $^{13}$C ($CDCl_3$) : 19,1 ; 36 ; 76,2 ; 111,9 ; 114.4 (d, J: 7,1), 115.5 (d, J: 24) ; 116,7 ; 118,8 (d, J: 7,4) ; 118,9 ; 121,3 (d, J: 23,1) ; 122,7 ; 125,2 ; 128,3 ; 129,2 ; 129,5 ; 137,8 ; 138,6 ; 142 ; 143,1 ; 148,7 ; 156,6 (d, J: 239) ; 161 ; 166,4.
LC/MS : temps de rétention : 3,23 min.
M+H. : 451,4.

**Préparation du composé 2**

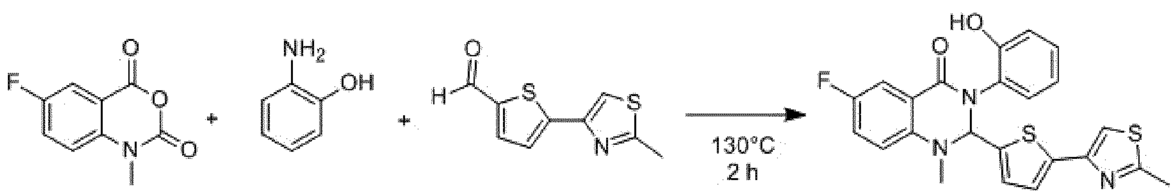


| Réactifs | éq. | M (g/mol) | n (mmol) | m (mg) | V (mL) |
|---|---|---|---|---|---|
| Anhydride 5-fluoro-N-méthylisatoïque | 1 | 195.03 | 1 | 195 | / |
| 2-aminophénol | 1 | 109.13 | 1 | 109 | / |
| 5-(2-méthyl-1,3-thiazol-4-yl)-2-thiophènecarbaldéhyde | 1 | 209.00 | 1 | 209 | / |
| Acide acétique | / | / | / | / | 2 |

Le mélange a été agité à 130°C pendant 2 heures. Le résidu a été dissous dans de l'EtOAc et lavé successivement avec une solution saturée de $NaHCO_3$ (trois fois), de la saumure (deux fois) et de l'eau (une fois). La phase organique a été séchée sur $MgSO_4$, filtrée et concentrée. Le résidu a été purifié par chromatographie (dépôt solide), éluée par un mélange cyclohexane/EtOAc : 95/5 -> 1/1, puis reprécipité par dissolution dans le minimum d'EtOAc et ajout de pentane (rendement : 25%).

RMN $^1$H (DMSO-d6) : 2,62 (s, 3H) ; 2,9 (s, 3H) ; 6,16 (s, 1H) ; 6,75 (td, 1H, J : 1,3, J : 7,7) ; 6,79 (dd, 1H, J : 4, J : 9) ; 6,93-7.01 (m, 3H) ; 7,15 (td, 1H, J : 1,7, J : 7,5) ; 7,28 (d, 1H, J : 3,6) ; 7,35 (td, 1H, J : 3,2, J : 8,8) ; 7,58 (dd, 1H, J : 3,1, J : 8,8) ; 7,71 (s, 1H).
RMN $^{13}$C (DMSO-d6) : 18,5 ; 35,5 ; 75,3 ; 112,7 ; 113,7 (d, J : 23,6) ; 115 (d, J : 7) ; 116,7 ; 117,8 (d, J : 7) ; 118,8 ; 121.1 (d, J : 23,1) ; 122,6 ; 126,2 ; 128,3 ; 128,7 ; 129,9 ; 137,6 ; 139 ; 143,3 ; 147,8 ; 152,6 ; 155,3 (d, J : 235) ; 159,5 ; 165,9.

IR : 1646 ; 1496 ; 1450 ; 1163 ; 806 ; 740
LC/MS : temps de rétention : 3,24 min.
M+H. : 452,4.

**Préparation du composé 3**

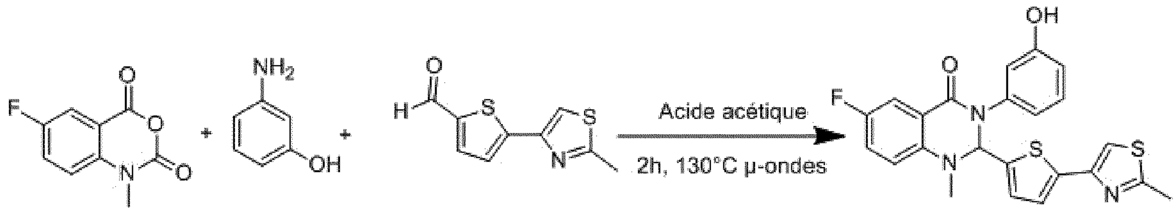


| Réactifs | Formule | Masse moléculaire | Masse (g) | Volume (mL) | n (mmol) |
|---|---|---|---|---|---|
| Anhydride N-méthyl-5-fluoroisatoïque | C6H4FNO3 | 195,15 | 0,195 | / | 1,0 |
| 3-aminophénol | C6H7NO | 109,13 | 0,109 | / | 1,0 |
| 5-(2-methyl-1,3-thiazol-4-yl)-2-thiophènecarbaldéhyde | C9H7NOS2 | 209,28 | 0,209 | / | 1,0 |
| Acide acétique | C2H4O2 | 60,05 | / | 2,00 | / |

Le mélange a été agité à 130°C pendant 2 heures. Le résidu a été dissous dans de l'EtOAc et la phase organique lavée successivement avec une solution saturée de $NaHCO_3$ et de l'eau distillée. La phase organique a été séchée sur $MgSO_4$, filtrée et concentrée sous vide. Le solide brun ainsi obtenu a été lavé avec une petite quantité d'EtOAc chaud pour donner une poudre jaune (17%).

RMN $^1$H (DMSO-d6) : 2,62 (s, 3H) ; 2,95 (s, 3H) ; 6,43 (s, 1H) ; 6,7- 6,78 (m, 3H) ; 6,81 (dd, 1H, J : 4,2, J : 9) ; 6,97 (d, 1H, J : 3.7) ; 7,20 (t, 1H, J : 8) ; 7,33 ( d, 1H, J : 3,6) ; 7,36 (dd, 1H, J : 3, J : 8,8) ; 7,61 (dd, 1H, J : 3, J : 8,8) ; 7,71 (s, 1H).
RMN $^{13}$C (DMSO-d6) : 18,5 ; 35,4 ; 76 ; 112,8 ; 113,7 ; 113,7 (d, J : 23) ; 114 ; 115,4 (d, J : 7) ; 116,8 ; 118 (d, J : 7) ; 121.3 (d, J : 23) ; 122,9 ; 128,2 ; 129,5 ; 137,7 ; 138,7 ; 140,8 ; 143,2 ; 147,7 ; 155,5 (d, J : 235) ; 157,6 ; 159,8 ; 166.
IR : 3161 (large) ; 1612 ; 1499 ; 1452 ; 1187 ; 1156 ; 795 ; 766 ; 730.
LC/MS : temps de rétention = 3,16 min.
M+H. : 452,3.

Le composé **7,** de formule suivante, est préparé de façon analogue au composé **2** ou **3.**

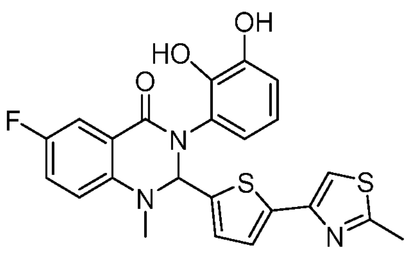
**7.**

**Préparation du composé 4**

**Synthèse du précurseur nitro 4'**

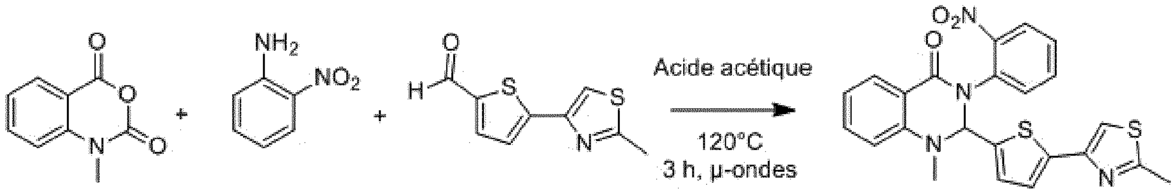


| Réactifs | éq. | M (g/mol) | n (mmol) | m (mg) | V (mL) |
|---|---|---|---|---|---|
| Anhydride *N*-méthylisatoïque | 1 | 177,04 | 1 | 177 | / |
| 2-nitroaniline | 1 | 138,13 | 1 | 138 | / |
| 5-(2-méthyl-1,3-thiazol-4-yl)-2-thiophènecarbaldéhyde | 1 | 209,00 | 1 | 209 | / |
| Acide acétique | / | / | / | / | 2 |

Le mélange a été agité à 120°C pendant 3 heures sous irradiation micro-ondes. Le résidu a été dissous dans de l'EtOAc et lavé successivement avec une solution saturée de $NaHCO_3$ (trois fois), de la saumure (deux fois) et de l'eau (une fois). La phase organique a été séchée sur $MgSO_4$, filtrée et concentrée sous vide. Le résidu a été purifié par chromatographie (dépôt solide), éluée par un mélange cyclohexane/EtOAc : 9/1 -> 1/1 (rendement : 49%).

**Synthèse du composé 4**

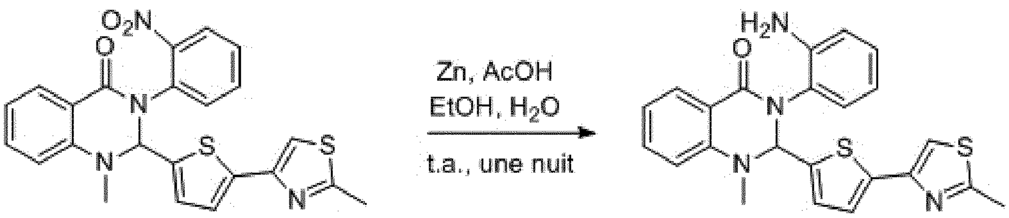


| Réactifs | éq. | M (g/mol) | n (mmol) | m (mg) | V (mL) |
|---|---|---|---|---|---|
| 4' | 1 | 462,08 | 0,79 | 365 | / |
| Zn | 30 | 65,39 | 30 | 1500 | / |
| EtOH | / | / | / | / | 10 |
| $H_2O$ | / | / | / | / | 1,8 |
| AcOH | / | / | / | / | 0,9 |

Le mélange a été agité une nuit à température ambiante, à l'abri de la lumière.

Le résidu obtenu a été purifié par chromatographie (dépôt solide), éluée par un mélange cyclohexane/EtOAc : 95/5 -> 0/1, puis reprécipité par dissolution dans le minimum d'EtOAc et ajout de pentane (rendement : 81%).
RMN $^1H$ (DMSO-d6) : 2,62 (s, 3H) ; 2,90 (s, 3H) ; 5,04 (s, 2H) ; 5,97 (s, 1H) ; 6,48 (td, 1H, J : 1,2, J : 7,3) ; 6,68 (dd, 1H, J : 1,2, J : 7,7) ; 6,77 (d, 1H, J : 8,2) ; 6 ; 6.87 (dd, 1H, J : 1, J : 8) ; 6,9-6,96 (m, 2H) ; 7,03 (td, 1H, J : 1,3, J :

8,4) ; 7,3 (d, 1H, J : 3,6) ; 7,48 (td, 1H, J : 1,5, J : 8,6) ; 7,72 (s, 1H) ; 7,88 (dd, 1H, J : 1,5, J : 7,7).
RMN $^{13}C$ (DMSO-d6) : 18,5 ; 34,9 ; 74,5 ; 112,7 ; 113 ; 115,9 ; 116,1 ; 117 ; 118,3 ; 122,6 ; 124,2 ; 128,1 ; 128,4 ; 128,7 ; 129,4 ; 134,2 ; 137,6 ; 138,7 ; 143,8 ; 143,8 ; 146,7 ; 147,8 ; 160,9 ; 165,9.
LC/MS : temps de rétention = 3,18 min.
M+H. : 433,4.

**Préparation du composé 5**

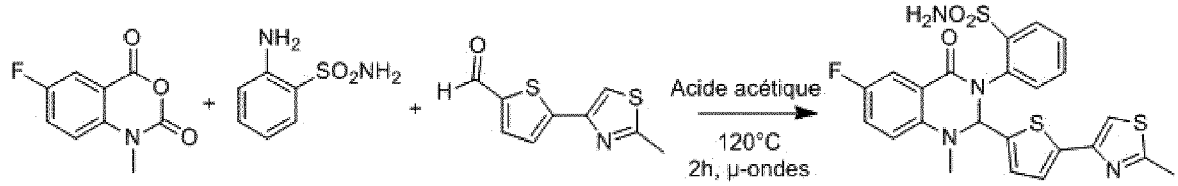


| Réactifs | éq. | M (g/mol) | n (mmol) | m (mg) | V (mL) |
|---|---|---|---|---|---|
| 2-aminobenzènesulfonamide | 1 | 172,20 | 0,4 | 69 | / |
| Anhydride 6-fluoro-*N*-méthylisatoïque | 1 | 195,03 | 0,4 | 78 | / |
| 5-(2-méthyl-1,3-thiazol-4-yl)-2-thiophènecarbaldéhyde | 1 | 209,28 | 0,4 | 84 | / |
| Acide acétique | / | / | / | / | 1 |

Le mélange a été agité à 120°C pendant 2 heures sous irradiation micro-ondes. Le résidu a été dissous dans du dichlorométhane et lavé successivement avec une solution saturée de $NaHCO_3$ (trois fois), de la saumure (deux fois) et de l'eau (une fois). La phase organique a été séchée sur $MgSO_4$, filtrée et concentrée sous vide. Le résidu a été purifié par chromatographie (dépôt solide), éluée par un mélange cyclohexane/EtOAc : 9/1 -> 0/1, puis recristallisé dans le dichlorométhane (rendement : 13%).

RMN $^1H$ (DMSO-d6) : 2,63 (s, 3H) ; 2,85 (s, 3H) ; 6,18 (s, 1H) ; 6,85 (dd, 1 H, J : 4,2, J : 9,1)] ; 6,89 (dd, 1H, J : 1,3, J : 7,4) ; 6,91 (d, 1H, J : 3.6) ; 7,32 (d, 1H, J : 7,6) ; 7,39 (td, 1H, J : 3,2, J : 8,7) ; 7,47 (s, 2H) ; 7,57-7,59 (m, 3H) ; 7,78 (s, 1H) ; 8,02 (dd, 1H, J : 1,9, J : 7,5).
RMN $^{13}C$ (DMSO-d6) : 18,5 ; 35,24 ; 76,7 ; 113,09 ; 113,7 (J : 23,6) 114,9 (J : 7) ; 116,6 (J : 7) ; 121,6 (J : 22,9) ; 122.6 ; 128.3 ; 128.84 ; 129.4 ; 132.1 ; 132.5 ; 136.1 ; 137.6 ; 138 ; 141.2 ; 143.3 ; 147.7 ; 155.1 (J : 235) ; 160.4 ; 166.
IR : 3175 ; 1653 ; 1496 ; 1474 ; 1450 ; 1357 ; 1342 ; 1160 ; 821 ; 810 ; 710.
LC/MS : temps de rétention = 3,20 min.
M+H. : 515,4.

**Préparation du composé 6 (hors invention)**

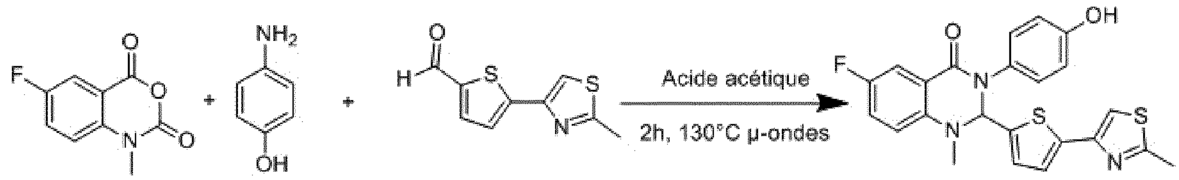

| Réactifs | Formule | Masse moléculaire | Masse (g) | Volume (mL) | n (mmol) |
|---|---|---|---|---|---|
| Anhydride N-méthyl-5-fluoroisatoïque | C6H4FNO3 | 195,15 | 0,180 | / | 0,92 |
| 4-aminophénol | C6H7NO | 109,13 | 0,100 | / | 0,92 |
| 5-(2-méthyl-1,3-thiazol-4-yl)-2-thiophènecarbaldéhyde | C9H7NOS2 | 209,28 | 0,192 | / | 0,92 |
| Acide acétique | C2H4O2 | 60,05 | / | 2,00 | / |

Le mélange a été agité à 130°C pendant 2 heures sous irradiation micro-ondes. Le résidu a été dissous dans de l'acétate d'éthyle et la phase organique a été lavée successivement avec une solution saturée de $NaHCO_3$ et de l'eau distillée. La phase organique a été séchée sur $MgSO_4$, filtrée et concentrée sous vide. Le solide brun ainsi obtenu a été lavé avec une petite quantité d'EtOAc chaud pour donner une poudre jaune (73%).

RMN $^1$H (DMSO-d6) : 2,60 (s, 3H) ; 2,89 (s, 3H) ; 6,32 (s, 1H) ; 6,74-6,77 (m, 3H) ; 6,92 (s large, 1H) ; 7,06 (d, 2H, J : 8,1) ; 7,3-7,35 (m, 2H) ; 7,57 (d, 1H, J : 6,6) ; 7,72 (s, 1H) ; 9,59 (s, 1H).
RMN $^{13}$C (DMSO-d6) : 18,5 ; 35,3 ; 76,4 ; 112,8 ; 113,7 (J : 24) ; 115,2 (J : 7) ; 115,4 ; 117,9 (J : 7) ; 121.2 (J : 23) ; 122,8 ; 128 ; 128,4 ; 131 ; 137,7 ; 138,5 ; 143,3 ; 147,7 ; 155,5 (J : 235) ; 156,3 ; 159,9 ; 166.
IR : 3216 (large) ; 1630 ; 1513 ; 1498 ; 1267 ; 1163 ; 888 ; 811 ; 737 ; 711.
LC/MS : temps de rétention = 3,33 min.
M+H. : 452,4.

**II. Mesure d'évaluation de l'activité protectrice des composés de l'invention vis-à-vis de la toxine de Shiga**

*Protocole et calcul de l'$IC_{50}$*

Les composés ont été testés soit sur cellules A549 (cellules épithéliales pulmonaires humaines) soit sur cellules HeLa (cellules de cancer utérin humain), contre les toxines de Shiga (Stx-1 et/ou Stx-2). Les cellules humaines sont cultivées à 37°C dans une atmosphère contenant 5% de $CO_2$ sur des flasques de culture de 150 $cm^2$ dans du milieu DMEM (Dulbecco's Modified Eagle Medium) contenant 100 U/mL de pénicilline et 100 μg/mL de streptomycine. Les cellules sont ensemencées à une densité de 50000 cellules par puits dans des plaques 96 puits à fond en scintillant solide Cytostar-T **(Figure 2).** Les cellules (100 μL dans du DMEM complet: DMEM + 10% de sérum de veau foetal, SVF) sont pré-incubées ou non avec les inhibiteurs (50 μL; différentes concentrations, pré-incubation de 3 h). Le milieu complet complémenté de toxine (50 μL, gamme de concentration variable) est ensuite ajouté à chaque puits. Après une incubation de 20 h, le milieu (200 μL) est éliminé et remplacé par un milieu DMEM sans leucine (Eurobio) contenant 10% de SVF et 0,5 μCi/mL de $^{14}$C-leucine (GE). Après une incubation de 7 h à 37°C, l'incorporation de radioactivité par les cellules est déterminée par lecture des plaques par un compteur à scintillation Wallac 1450 Microbeta trilux (PE).

Comme ces toxines bloquent la synthèse des protéines, les cellules affectées ne sont plus capables d'incorporer la leucine radiomarquée. Par contre, les cellules traitées par des inhibiteurs synthétisent toujours des protéines et incorporent donc l'acide aminé radiomarqué. Comme les cellules concentrent le radioélément suffisamment près du fond du puits, cela entraine une excitation du scintillant contenu dans les plaques et conduit à l'émission de photons détectée par le compteur à scintillation (mesure en coups par minutes, cpm). Ces données sont ensuite exprimées en pourcentage de synthèse de protéine par les cellules. Les courbes de cytotoxicité peuvent ainsi être tracées **(Figure 2)** sans inhibiteur (ronds blancs) ou en présence d'un inhibiteur (ronds noirs). L'analyse des données par régression non linéaire permet d'estimer l'$EC_{50}$, soit la concentration efficace pour laquelle on observe 50 % d'assimilation de leucine radioactive ce qui correspond à 50% de cellules viables. Plus la valeur de l'$EC_{50}$ est élevée, plus la protection cellulaire est importante car il faut alors une concentration plus élevée de toxine pour générer la même cytotoxicité. Il est ainsi possible de déterminer l'efficacité des inhibiteurs en calculant le ratio R des $EC_{50}$ **(Figure 2).** Plus la valeur est élevée (> 1), plus la protection des cellules est importante.

Six courbes de cytotototoxicité sont obtenues en l'absence puis en présence de concentrations croissantes d'inhibiteur. Pour chaque concentration (C) d'inhibiteur, un pourcentage de protection est déterminé à partir de la valeur de R calculée par le logiciel Prism avec Rmax correspondant à la valeur maximale de R de la série:

$$\%\ protection = [(R-1)/(Rmax-1)] * 100$$

L'$IC_{50}$ est ensuite calculée, à l'aide du logiciel Prism par régression non linéaire, à partir d'un graphique où sont reportés pour chaque concentration de composé le pourcentage de protection cellulaire correspondant (*cf.* **Figure 3**).

$IC_{50}$ correspond à la concentration de composé donnant 50% de son pouvoir inhibiteur. Plus cette concentration est faible, plus l'inhibiteur est puissant.

***Résultats***

| Composé | $IC_{50}$(nM) |
|---|---|
| Retro-2.1 (Contrôle) | 100,0 |
| **4** | 78,6 |
| **1** | 14,2 |
| **2** | 20,0 |
| **3** | 55,0 |
| **5** | 35,0 |
| **7** | 41,0 |
| **6** (hors invention) | 483,0 |

Le composé Retro-2.1 est de formule suivante :

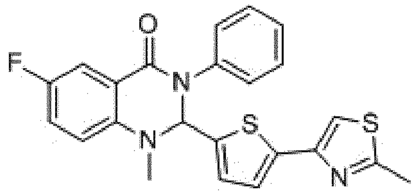
.

**CONCLUSION**

Ces résultats montrent que les composés testés sont tous des inhibiteurs de l'effet cellulaire de la toxine Shiga plus puissants que le composé Retro-2.1 (contrôle hors invention, dans lequel la dihydroquinazolinone porte un phényle non substitué), ainsi que le composé **6** (hors invention, dans lequel la dihydroquinazolinone porte un phényle substitué en para par un groupe hydroxyle).

**III. Mesure d'évaluation de l'activité protectrice des composés de l'invention vis-à-vis de la ricine**

Le protocole et le calcul de l'$IC_{50}$ des composés de l'invention est analogue à celui présenté au paragraphe II, en prenant pour toxine la ricine.

**IV. Mesure d'évaluation de l'activité protectrice des composés de l'invention vis-à-vis de virus utilisant le transport rétrograde et/ou dépendant de la syntaxine 5, notamment de virus entrant dans les cellules par endocytose.**

Des cellules comme indiquées ci-après ont été ensemencées à une densité de 50 % dans des plaques de 96 puits dans un milieu MEM additionné de 10 % de sérum foetal bovin et incubées à 37 °C, 5 % de $CO_2$. On a laissé les cellules reposer pendant la nuit. Les composés à tester ont été dissous dans le DMSO à 20 mM chacun. Le jour suivant, chaque composé a été dilué 100 fois dans du MEM avec du sérum, dilué en série (à 5 concentrations) et ajouté en volume égal au milieu cellulaire dans chaque puits. Après 1 heure de préincubation, le virus a été ajouté à une multiplicité d'infection (MOI) telle qu'indiquée ci-après. Les virus utilisés sont notamment des virus recombinant qui codent une protéine fluorescente verte (Towner et al. 2005. Virology 332:20-7 ; ou virus ANCHOR fournis par NEOVIRTECH). Ces virus sont capables de se répliquer et présentent une pathogenèse normale chez l'animal. Les cellules ont ensuite été fixées à l'aide de formol à 10 % pendant le temps indiqué ci-après. Les noyaux des cellules ont été colorés avec du

DAPI selon des méthodes habituelles. L'infection a ensuite été analysée en photographiant chaque puits à l'aide d'un microscope épifluorescent. Les cellules totales et infectées ont été comptées en comptant respectivement les cellules colorées par DAPI et les cellules fluorescentes vertes à l'aide du logiciel Cell Profiler. La fraction de cellules infectées a été calculée en divisant le nombre de cellules fluorescentes vertes par le nombre total de cellules. L'évaluation de la $CI_{50}$ a été effectuée en fonction du nombre de cellules à l'aide du logiciel Combenefit (Di Veroli et al. Bioinformatics 2016, 32(18), 2866-8).

Conditions d'infection et de fixation :

- Cellules MRC5 avec hCMV-ANCHOR, MOI 1 ; fixation à 6 jours ;
- Cellules HeLa avec VACV-ANCHOR, MOI 0,1 ; fixation à 48 heures ;
- Cellules RK13 avec Myxomavirus T1-ANCHOR, MOI 0,05 ; fixation à 6 jours ;
- Cellules Hep2 avec RSV ; MOI 0,2 ; fixation à 72 heures.

Toxicité

Des concentrations en composé de l'invention de 100 μM à 0,2 μM ont été testées (2 fois). Les cellules telles que décrites ci-dessus ont été fixées 72 heures après ajout des composés de l'invention. Le nombre de cellules comptées par puits est normalisé sur le nombre de cellules comptées dans les puits correspondants traités uniquement avec du DMSO. L'évaluation de la $CC_{50}$ a été effectuée en fonction du nombre de cellules à l'aide du logiciel Combenefit précité.

Résultats

Les résultats obtenus sont consignés dans le tableau suivant :

| | | **Retro-2.1** | **2** | **1** | **6 (hors invention)** |
|---|---|---|---|---|---|
| **HeLa / VacV** | **CC50 (nM)** | > 50 000 | 32 300 | >50 000 | 20 400 |
| | **CI50 (nM)** | 37,8 | 3,61 | 1,05 | 628 |
| | **Index de sélectivité** | 1 323 | 8 947 | 47 619 | 32 |
| | | | | | |
| **RK13 / MyxV T1** | **CC50 (nM)** | 3 350 | ND | 7 460 | 12 900 |
| | **CI50 (nM)** | 74,5 | 14,2 | 1,44 | 5 800 |
| | **Index de sélectivité** | 45 | ND | 5 181 | 2 |
| | | | | | |
| **MRC5 / hCMV** | **CC50 (nM)** | > 50 000 | 21 000 | 21 500 | 16 700 |
| | **CI50 (nM)** | 11,6 | < 1 | < 1 | 281 |
| | **Index de sélectivité** | 4 310 | 21 000 | 21 500 | 59 |
| | | | | | |
| **Hep2 / RSV** | **CC50 (nM)** | > 50 000 | 37 600 | > 50 000 | 25 600 |
| | **CI50 (nM)** | 162 | 20 | ND | 2200 |
| | **Index de sélectivité** | 309 | 1880 | ND | 12 |

**V. Mesure d'évaluation de l'activité protectrice des composés de l'invention vis-à-vis d'une bactérie utilisant le transport rétrograde et/ou dépendant de la syntaxine 5, notamment une bactérie entrant dans les cellules par endocytose**

Des cellules HeLa229 sont prétraitées avec le composé de l'invention à évaluer à des concentrations de 25, 50 et 75 μM pendant 30 minutes jusqu'à ce que la bactérie soit ajoutée aux cellules.

Les cellules présentant une infection primaire sont lysées 24 h après l'infection (hpi).

Des cellules traitées avec ledit composé sont également lysées 48 h après l'infection et le lysat obtenu est utilisé pour infecter de nouvelles cellules HeLa229, lesquelles sont lysées 24 h après l'infection et analysées avec les

échantillons d'infection primaire par immunoblot. La croissance de la bactérie est détectée à l'aide d'anticorps ad hoc et l'actine est utilisée comme contrôle.

## VI. Synthèse de prodrogues selon l'invention

Des prodrogues selon la présente invention ont été synthétisées comme suit :

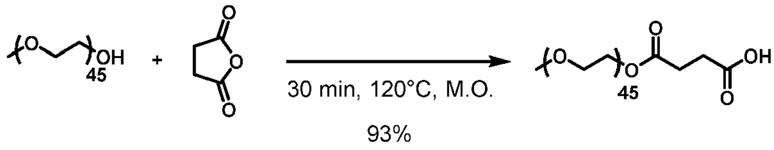


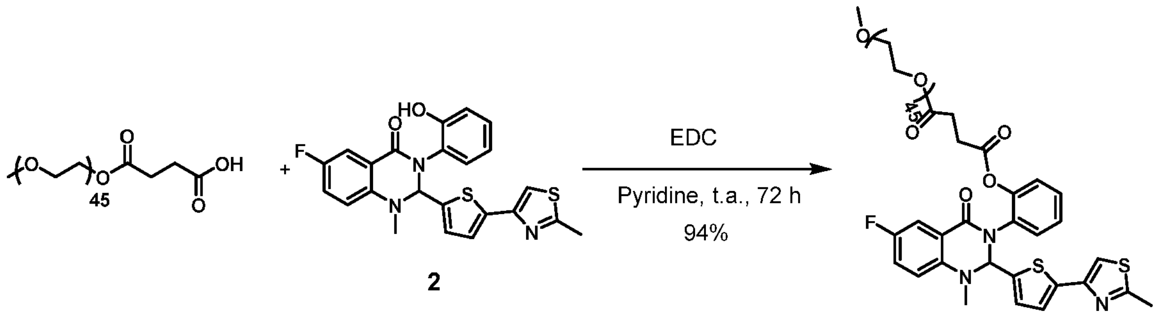


2

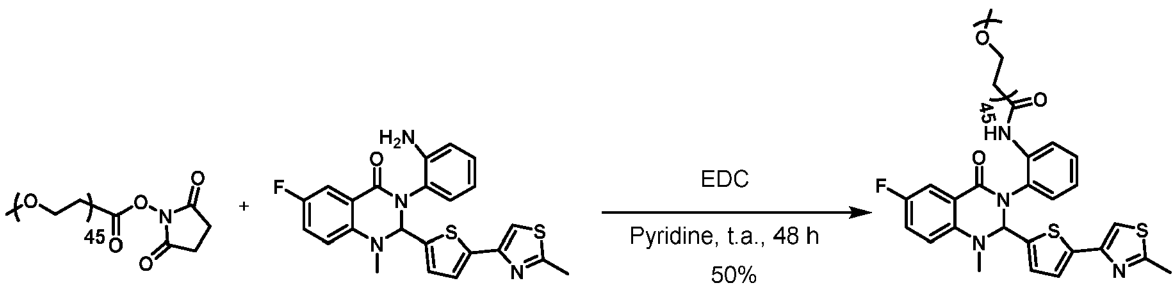


1

Les composés ainsi obtenus ont une excellente solubilité dans l'eau (>700 mg/mL, administration possible en minipompes, perfusion, injection i.v.). Leur clivage par les amidases et/ou les estérases plasmatiques est rapide (par exemple avec un $t_{1/2}$ de 2,6 heures dans du plasma de souris à 37°C).

D'autres prodrogues, sous la forme de polymères thermogélifiants ont été obtenus comme suit :

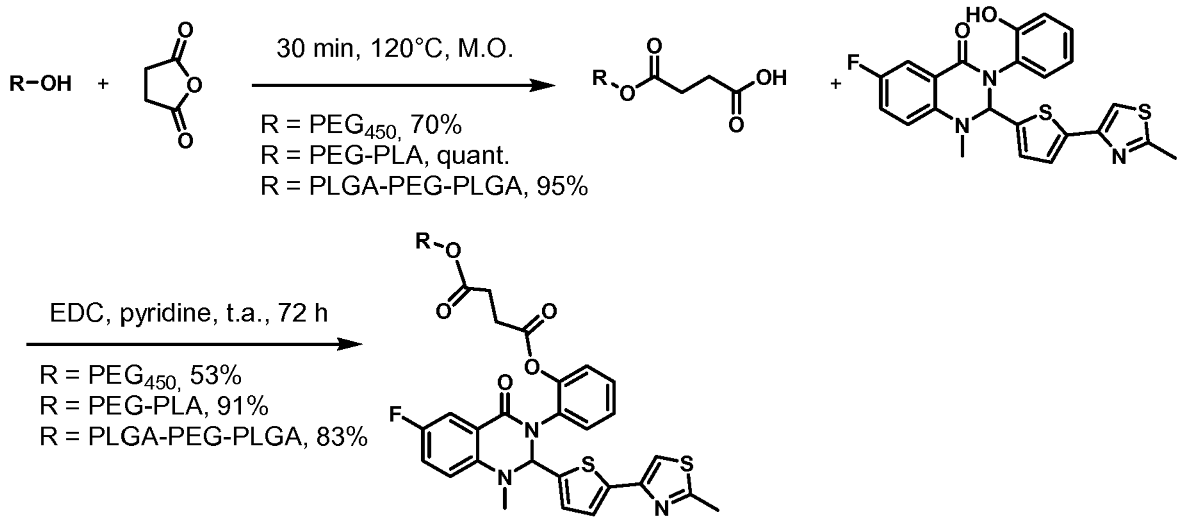


Ou encore comme suit :

(Composé **2**)-PLGA1036-PEG1450-PLGA1036-(Composé **2**) (Mn = 4393 g/mol) ou (Composé **2**)-C(=O)-$(CH_2)_2$-C(=O)-PLGA1036-PEG1450-PLGA1036-C(=O)-$(CH_2)_2$-C(=O)-(Composé **2**), en particulier de formule suivante :

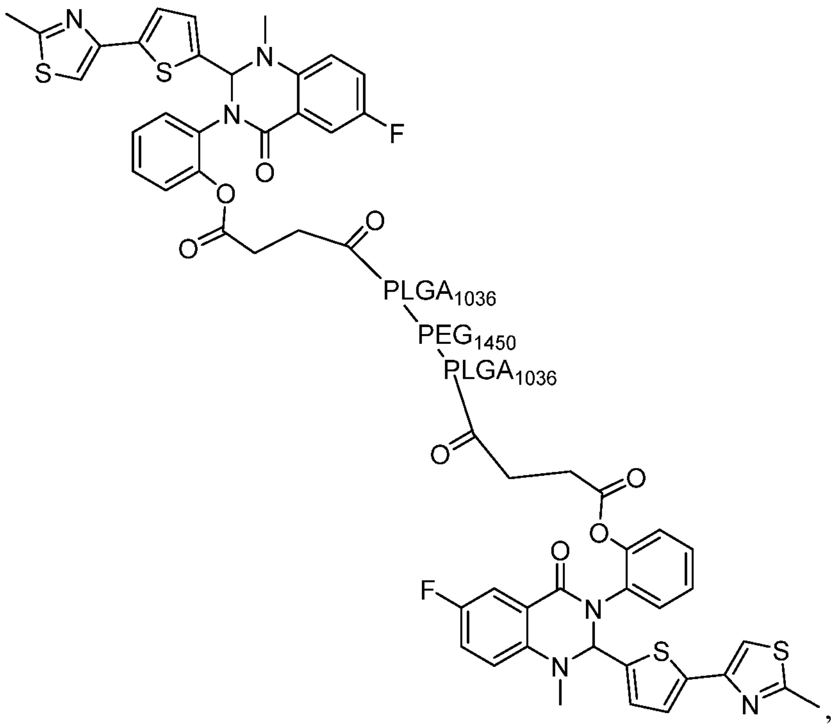


soluble dans l'eau et aux propriétés de thermogélation.

Le PLGA1036-PEG1450-PLGA1036 est par exemple obtenu comme suit :

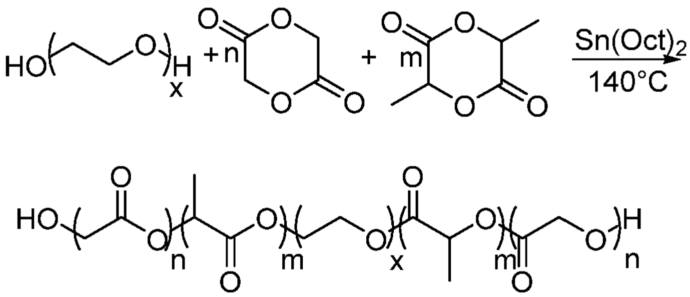


Le poly(éthylène glycol) 1450 (3,00g - 2,07mmol) est placé dans un schlenk, chauffé à 100°C dans un bain d'huile. Le schlenk est placé sous vide pendant 2h sous agitation. Le glycolide (0,6g - 5,13mmol) et le D,L-Lactide (2,64g - 18,3 mmol) sont ajoutés au schlenk et le mélange est chauffé à 130°C jusqu'à fusion complète des solides. Le $Sn(Oct)_2$ (une goutte) est ajouté. Le mélange est chauffé 20h à 130°C sous agitation, sous azote. Le mélange est ramené à TA puis dissous dans 20mL d'acétone. La solution est ajoutée goutte à goutte à de l'eau distillée à 4°C sous agitation. Le mélange est agité à 4°C jusqu'à solubilisation complète du polymère puis le mélange est chauffé à 80°C. Le polymère est récupéré par décantation. Cette séquence est répétée puis le solide est dissous dans du dichlorométhane. La phase organique est séchée sur $MgSO_4$, filtrée et évaporée pour donner une gomme translucide qui est séchée sous vide à 40°C pendant 15h.

Produit purifié : 3,8g (49%).
Masse molaire (PLA)=1707 g/mol ; Masse molaire (PGA)=365 g/mol, correspondant à un composé PLGA1036-PEG1450-PLGA1036 (Mn=3522g/mol ; LA/GA=3,8).

## Revendications

**1.** Composé de formule générale (I) :

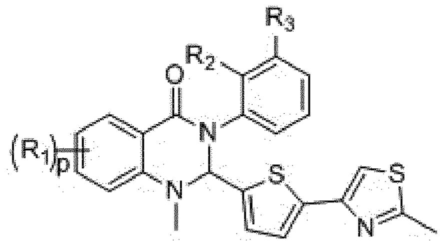
(I)

où :

- p est égal à 1, 2 ou 3 ;
- Ri représente à chaque occurrence, de façon indépendante, un atome d'hydrogène, un atome d'halogène, un radical alcoxy de 1 à 3 atomes de carbone, notamment un groupe méthoxy, $-NO_2$, ou $-NH_2$ ;
- $R_2$ et $R_3$ représentent indépendamment l'un de l'autre un groupe choisi parmi H, -OH, $-OR_4$, $-NH_2$, $-NHR_5$, $-SO_2-NH_2$, $-SO_2-NH-R_6$, à la condition que l'un au moins de $R_2$ et $R_3$ ne représente pas H ;
- $R_4$, $R_5$ et $R_6$ représentent indépendamment les uns des autres un groupe de formule (II) $-L-(X)_i-(PEG)-(Y)_j-Z$, où :

    - i et j représentent indépendamment l'un de l'autre 0 ou 1 ;
    - L représente -C(=O)- ou $-C(=O)-(CH_2)_k-C(=O)-$, avec k étant égal à 1, 2 ou 3, en particulier 2 ;
    - X et Y représentent indépendamment l'un de l'autre un poly(acide lactique) ou un poly(acide lactique-co-acide glycolique) ;
    - PEG représente un poly(éthylène glycol) ;

- Z représente un groupe choisi parmi H, un alkyle en $C_1$ à $C_3$, -OH, un O-alkyle en $C_1$ à $C_3$, ou $-L-R_e$, où L est défini tel que précédemment et $R_e$ est un résidu de formule (I) relié audit groupe $-L-(X)_i-(PEG)-(Y)_j-$ défini

précédemment par l'intermédiaire de son groupe $R_2$ ou $R_3$, ledit groupe $R_2$ ou $R_3$ étant -OH, $-NH_2$ ou $-SO_2-NH_2$;

ainsi que les formes stéréoisomères, les mélanges de formes stéréoisomères ou leurs sels pharmaceutiquement acceptables.

**2.** Composé de formule générale (I) selon la revendication 1, dans lequel $R_3$ représente un atome d'hydrogène.

**3.** Composé de formule générale (I) selon l'une quelconque des revendications précédentes, dans lequel $R_2$ représente un atome d'hydrogène.

**4.** Composé de formule générale (I) selon l'une quelconque des revendications précédentes, dans lequel p est égal à 1.

**5.** Composé de formule générale (I) selon l'une quelconque des revendications précédentes, dans lequel $R_1$ représente un atome d'halogène, en particulier un atome de fluor.

**6.** Composé de formule générale (I) selon la revendication 1, de formule (Ia) suivante :

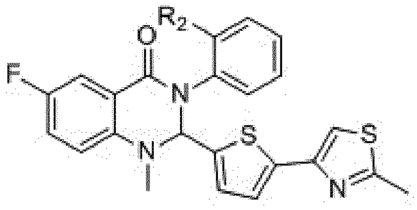

(Ia).

**7.** Composé de formule générale (I) selon la revendication 1, choisi parmi :

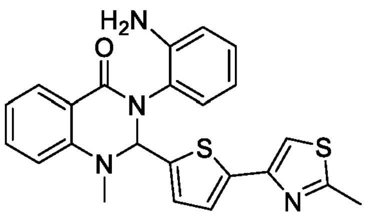

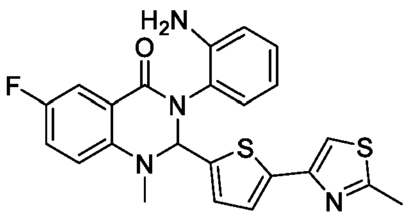

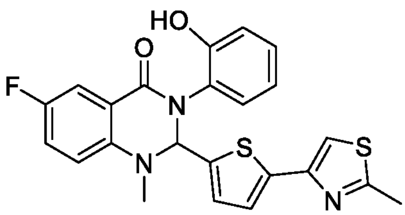

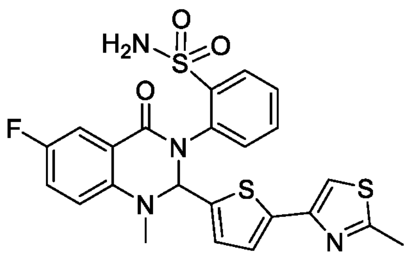

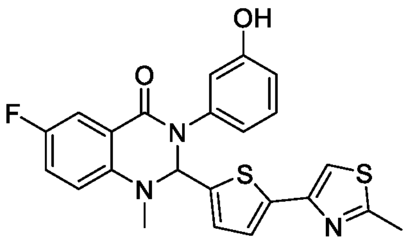

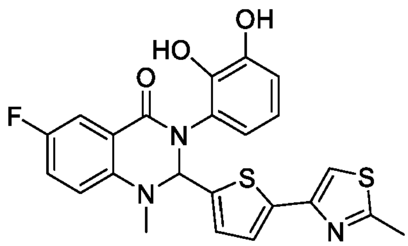

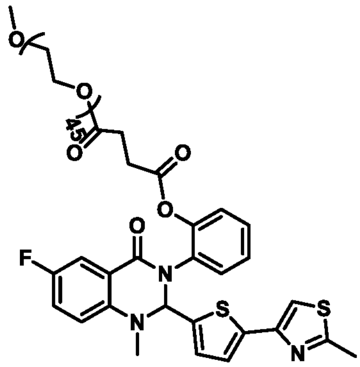 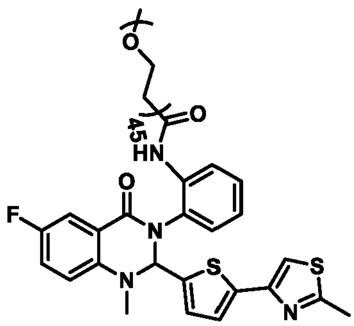

R = $PEG_{450}$
R = PEG-PLA
R = PLGA-PEG-PLGA
en particulier $PLGA_{1036}$-$PEG_{1450}$-$PLGA_{1036}$

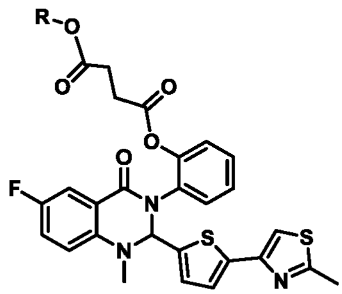

et

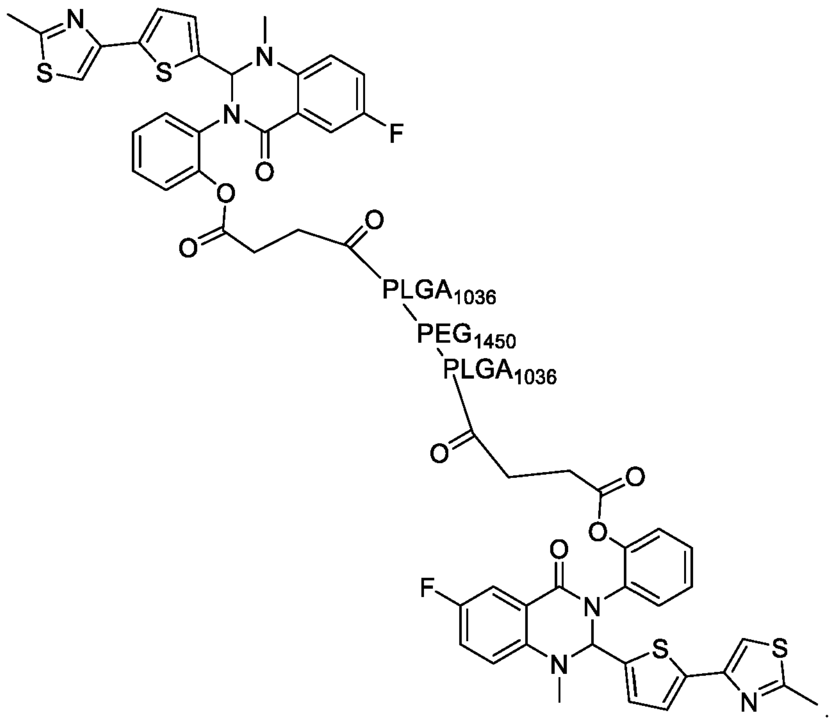

**8.** Composé de formule générale (I) tel que défini dans l'une quelconque des revendications 1 à 7, pour son utilisation pour la prévention et/ou le traitement des désordres induits par les toxines à mode d'action intracellulaire utilisant le transport rétrograde choisies parmi la ricine, la toxine de Shiga et les toxines Shiga-like (Stxs) produites par *Shigella dysenteriae* (Stx) et *E. coli,* la toxine cholérique, la toxine pertussique, la cytotoxine subtilase et l'entérotoxine thermolabile, par les virus utilisant le transport rétrograde et/ou dépendant de la syntaxine 5 pour infecter les cellules, choisis parmi les virus pox, les cytomégalovirus, les adénovirus et les virus adéno-associés, les polyomavirus, les papillomavirus, les filovirus, les entérovirus, les virus de l'herpès, les virus du genre *Arenavirus,* les virus de la grippe, et les pneumovirus, les bactéries utilisant le transport rétrograde et/ou dépendant de la syntaxine 5 pour

infecter les cellules, choisies parmi les bactéries de l'ordre des Chlamydiales ou *Symkania,* ou par les parasites intracellulaires.

**9.** Composé pour son utilisation selon la revendication 8, **caractérisé en ce que** lesdites toxines à mode d'action intracellulaire sont choisies parmi la ricine, la toxine de Shiga et les toxines Shiga-like (Stxs) produite par *Shigella dysenteriae* (Stx) et *E. coli* (Stx1 et Stx2), la toxine cholérique (Ctx de *Vibrio cholerae* responsable du choléra), la toxine pertussique (*Bordetella pertussis* agent de la coqueluche), la cytotoxine subtilase et l'entérotoxine thermolabile (*E. coli*).

**10.** Composé pour son utilisation selon la revendication 8, **caractérisé en ce que** lesdits virus sont les virus pox, notamment le virus de variole, le virus monkeypox, le virus Vaccinia et les leporipoxvirus, en particulier le virus de la myxomatose, les cytomégalovirus, les virus adéno-associés, en particulier de sérotype 2, les polyomavirus, notamment le polyomavirus JC et le polyomavirus BK, les papillomavirus, les filovirus, notamment les virus Ebola et le virus Marburg, les entérovirus, notamment l'entérovirus 71, les virus de l'herpès, notamment le virus Herpes simplex de type 2, les virus du genre *Arenavirus,* notamment le virus de la chorioméningite lymphocytaire, les virus de la grippe, notamment les influenzavirus A, les pneumovirus, notamment le virus respiratoire syncytial.

**11.** Composition pharmaceutique ou médicament comprenant au moins un composé de formule générale (I) tel que défini dans l'une quelconque des revendications 1 à 7 en tant que principe actif, et un véhicule pharmaceutiquement acceptable, ladite composition pharmaceutique ou ledit médicament étant notamment adapté à une administration par les voies aérienne, orale, parentérale ou locale.

**Patentansprüche**

**1.** Verbindung der allgemeinen Formel (I):

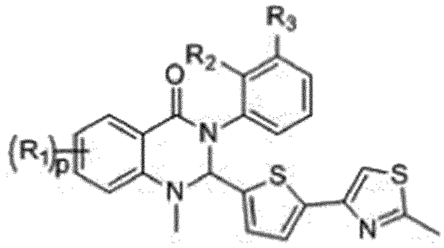

(I)

worin:

- p gleich 1, 2 oder 3 ist;
- $R_1$ bei jedem Auftreten unabhängig ein Wasserstoffatom, ein Halogenatom, einen Alkoxyrest mit 1 bis 3 Kohlenstoffatomen, insbesondere eine Methoxygruppe, $-NO_2$ oder $-NH_2$ darstellt;
- $R_2$ und $R_3$ unabhängig voneinander für eine Gruppe stehen, die ausgewählt ist aus H, -OH, $-OR_4$, $-NH_2$, $-NHR_5$, $-SO_2-NH_2$, $-SO_2-NH-R_6$, mit der Maßgabe, dass mindestens einer von $R_2$ und $R_3$ nicht H darstellt;
- $R_4$, $R_5$ und $R_5$ unabhängig voneinander eine Gruppe der Formel (II) $-L-(X)_i-(PEG)-(Y)_j-Z$, worin:

  - i und j unabhängig voneinander 0 oder 1 darstellen;
  - L -C(=O)- oder $-C(=O)-(CH_2)_k-C(=O)-$ darstellt, wobei k gleich 1, 2 oder 3 ist, insbesondere 2;
  - X und Y unabhängig voneinander eine Poly(milchsäure) oder eine Poly(milchsäure-co-glykolsäure) darstellen;
  - PEG ein Poly(ethylenglycol) darstellt;

- Z eine Gruppe darstellt, die ausgewählt ist aus H, ein $C_1$- bis $C_3$-Alkyl, -OH, ein $C_1$- bis $C_3$-O-Alkyl oder $-L-R_e$, worin L wie vorstehend definiert ist und $R_e$ ein Rest der Formel (I) ist, der mit der vorstehend definierten Gruppe $-L-(X)_i(PEG)-(Y)_j-$ über ihre Gruppe $R_2$ oder $R_3$ verbunden ist, wobei die Gruppe $R_2$ oder $R_3$ -OH, $-NH_2$ oder $-SO_2-NH_2$ ist;

sowie die stereoisomere Formen, die Gemische von stereoisomeren Formen oder deren pharmazeutisch verträgliche Salze.

**2.** Verbindung der allgemeinen Formel (I) nach Anspruch 1, wobei $R_3$ ein Wasserstoffatom darstellt.

**3.** Verbindung der allgemeinen Formel (I) nach einem der vorstehenden Ansprüche, wobei $R_2$ ein Wasserstoffatom darstellt.

**4.** Verbindung der allgemeinen Formel (I) nach einem der vorstehenden Ansprüche, wobei p gleich 1 ist.

**5.** Verbindung der allgemeinen Formel (I) nach einem der vorstehenden Ansprüche, wobei $R_1$ ein Halogenatom, insbesondere ein Fluoratom, darstellt.

**6.** Verbindung der allgemeinen Formel (I) nach Anspruch 1 mit der folgenden Formel (Ia):

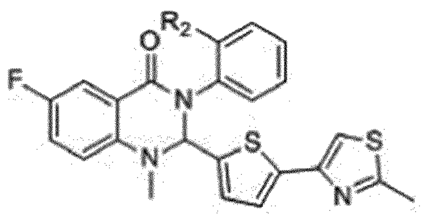

(Ia).

**7.** Verbindung der allgemeinen Formel (I) nach Anspruch 1, ausgewählt aus:

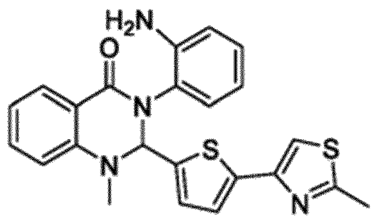

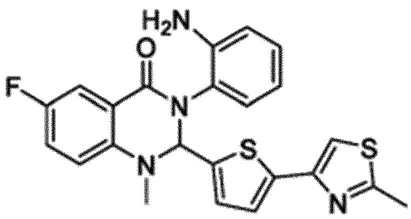

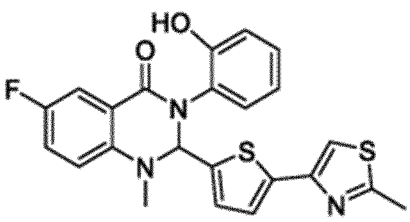

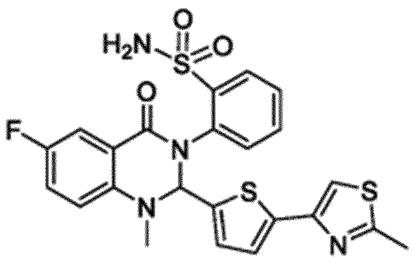

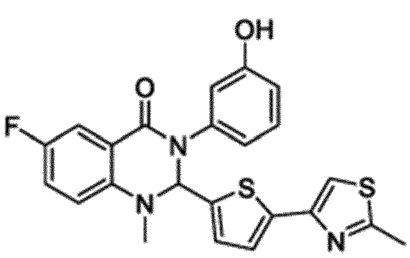

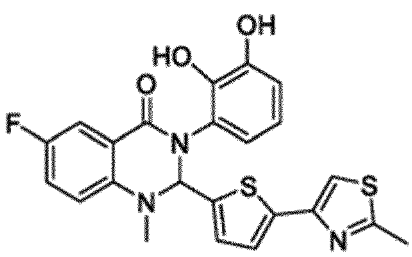

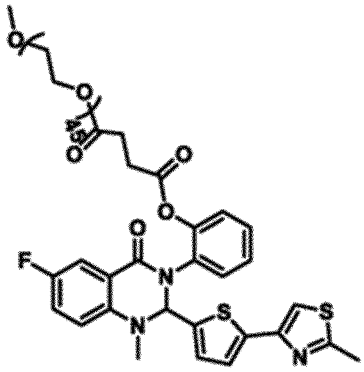

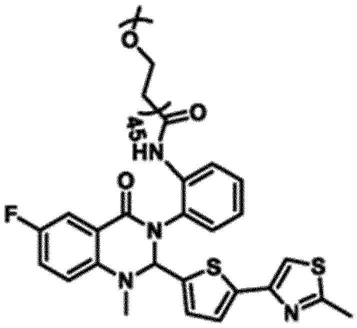

R = $PEG_{450}$
R = PEG-PLA
R = PLGA-PEG-PLGA
**insbesondere $PLGA_{1036}$-$PEG_{1450}$-$PLGA_{1036}$**

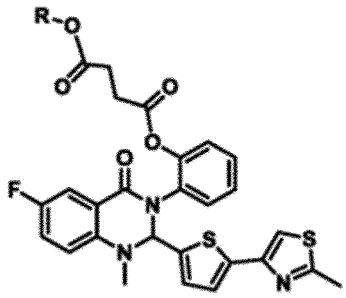

und

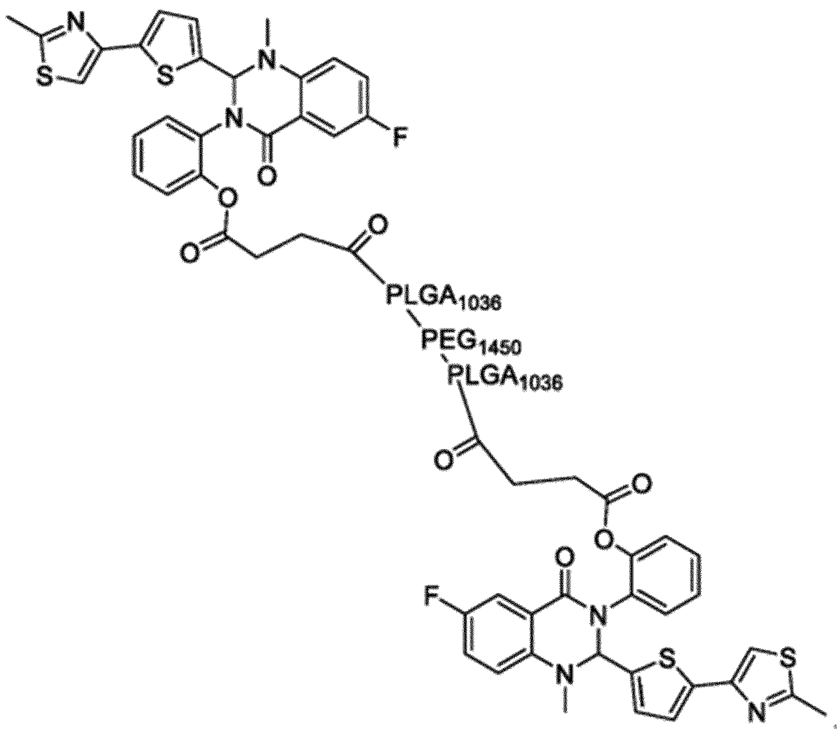

**8.** Verbindung der allgemeinen Formel (I), wie in einem der Ansprüche 1 bis 7 definiert, für ihre Verwendung zur Vorbeugung und/oder Behandlung von Störungen, die durch Toxine mit intrazellulärer Wirkungsweise unter Verwendung des retrograden Transports induziert werden, ausgewählt aus Rizin, Shiga-Toxin und shigaähnlichen Toxinen (Stxs), die von *Shigella dysenteriae* (Stx) und *E. coli,* Cholera-Toxin, Pertussis-Toxin, Subtilase-Zytotoxin und thermolabilem Enterotoxin, durch Viren, die den retrograden und/oder Syntaxin-5-abhängigen Transport zur Infektion von Zellen nutzen, ausgewählt aus Pox-Viren, Cytomegaloviren, Adenoviren und adeno-assoziierten Viren, Polyomaviren, Papillomaviren, Filoviren, Enteroviren, Herpesviren, Viren der Gattung *Arenavirus,* Grippeviren und

Pneumoviren, Bakterien, die den retrograden und/oder Syntaxin-5-abhängigen Transport zur Infektion von Zellen nutzen, ausgewählt aus Bakterien der Ordnung Chlamydiales oder *Symkania,* oder durch intrazelluläre Parasiten.

**9.** Verbindung zur Verwendung nach Anspruch 8, **dadurch gekennzeichnet, dass** die Toxine mit intrazellulärer Wirkungsweise ausgewählt sind aus Rizin, Shiga-Toxin und shigaähnlichen Toxinen (Stxs), die von *Shigella dysenteriae* (Stx) und *E. coli* (Stx1 und Stx2), Cholera-Toxin (Ctx von *Vibrio cholerae,* Verursacher von Cholera), Pertussis-Toxin (*Bordetella pertussis,* Erreger des Keuchhustens), Subtilase-Zytotoxin und thermolabilem Enterotoxin (*E. coli*).

**10.** Verbindung für ihre Verwendung nach Anspruch 8, **dadurch gekennzeichnet, dass** die Viren Pockenviren, insbesondere das Variola-Virus, das Monkeypox-Virus, das Vaccinia-Virus und die Leporipoxviren, insbesondere das Myxomatose-Virus, Cytomegaloviren, adeno-assoziierte Viren, insbesondere vom Serotyp 2, Polyomaviren, insbesondere das Polyomavirus JC und das Polyomavirus BK, Papillomaviren, Filoviren, insbesondere Ebola- und Marburg-Viren, Enteroviren, insbesondere Enterovirus 71, Herpesviren, insbesondere Herpes-simplex-Virus Typ 2, Viren der Gattung *Arenavirus,* insbesondere das lymphozytäre Choriomeningitis-Virus, Influenzaviren, insbesondere Influenzavirus A, Pneumoviren, insbesondere das Respiratory-Syncytial-Virus sind.

**11.** Pharmazeutische Zusammensetzung oder Medikament, umfassend mindestens eine Verbindung der allgemeinen Formel (I), wie in einem der Ansprüche 1 bis 7 definiert, als Wirkstoff und einen pharmazeutisch annehmbaren Träger, wobei die pharmazeutische Zusammensetzung oder das Medikament insbesondere an eine Verabreichung über die Luftwege, orale, parenterale oder lokale Wege angepasst ist.

## Claims

**1.** A compound of general formula (I):

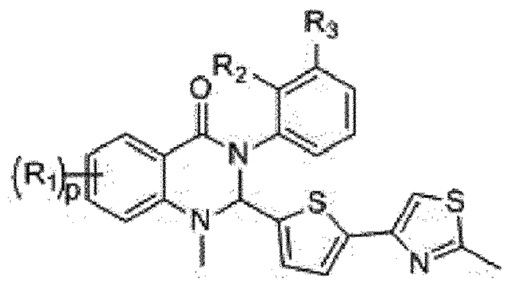

(I)

wherein:

- p represents 1, 2 or 3 ;
- $R_1$ represents at each occurrence, independently, a hydrogen atom, a halogen atom, an alkoxy radical of 1 to 3 carbon atoms, in particular methoxy, $-NO_2$ or $-NH_2$ group;
- $R_2$ and $R_3$ represent, independently of one another, a group selected from H, -OH, $-OR_4$, $-NH_2$, $-NHR_5$, $-SO_2-NH_2$, $-SO_2-NH-R_6$, with the proviso that at least one of $R_2$ and $R_3$ does not represent H ;
- $R_4$, $R_5$ and $R_5$ represent, independently of one another, a group of formula (II) $-L-(X)_i-(PEG)-(Y)_j-Z$, wherein:
    - i and j represent, independently of one another, 0 or 1;
    - L represents -C(=O)- or $-C(=O)-(CH_2)_k-C(=O)-$, with k being equal to 1, 2 or 3, in particular 2 ;
    - X and Y represent, independently of one another, a poly(lactic acid) or a poly(lactic acid-co-glycolic acid);
    - PEG represents a poly(ethylene glycol);
    - Z represents a group selected from H, $C_1$ to $C_3$ alkyl, -OH, O-$C_1$ to $C_3$ alkyl, or $-L-R_e$, wherein L is defined as above and $R_e$ is a residue of formula (I) linked to said $-L-(X)_i-(PEG)-(Y)_j-$ group defined above via its $R_2$ or $R_3$ group, said $R_2$ or $R_3$ group being -OH, $-NH_2$ or $-SO_2-NH_2$;

as well as stereoisomeric forms, mixtures of stereoisomeric forms or pharmaceutically acceptable salts thereof.

**2.** The compound of general formula (I) according to claim 1, in which $R_3$ represents a hydrogen atom.

3. The compound of general formula (I) according to any one of the preceding claims, in which $R_2$ represents a hydrogen atom.

4. The compound of general formula (I) according to any one of the preceding claims, in which p is equal to 1.

5. The compound of general formula (I) according to any one of the preceding claims, in which $R_1$ represents a halogen atom, in particular a fluorine atom.

6. The compound of the general formula (I) according to claim 1, of the following formula (Ia):

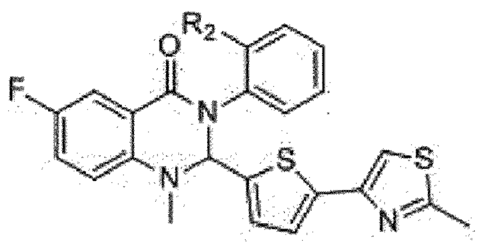

(Ia).

7. The compound of general formula (I) according to claim 1, selected from:

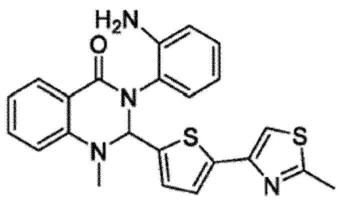

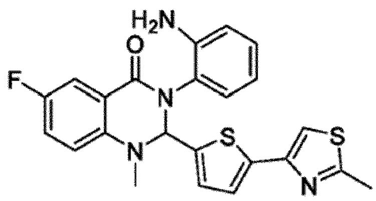

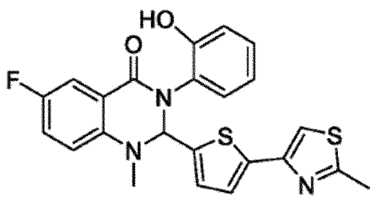

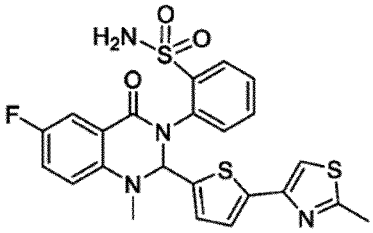

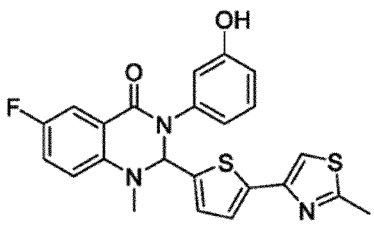

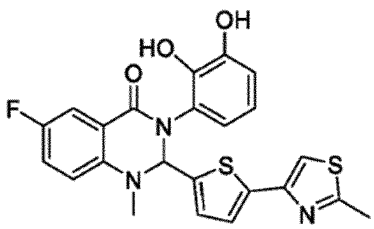

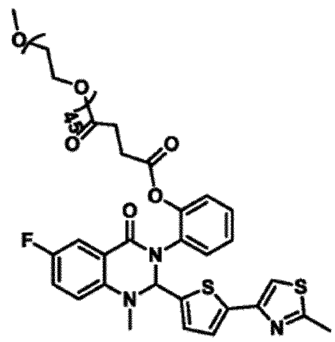

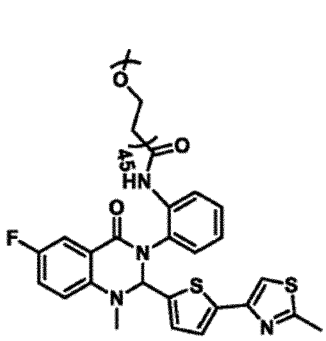

R = $PEG_{450}$
R = PEG-PLA
R = PLGA-PEG-PLGA
in particular $PLGA_{1036}$-$PEG_{1450}$-$PLGA_{1036}$

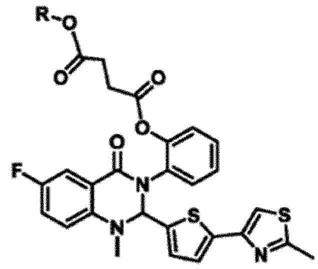

and

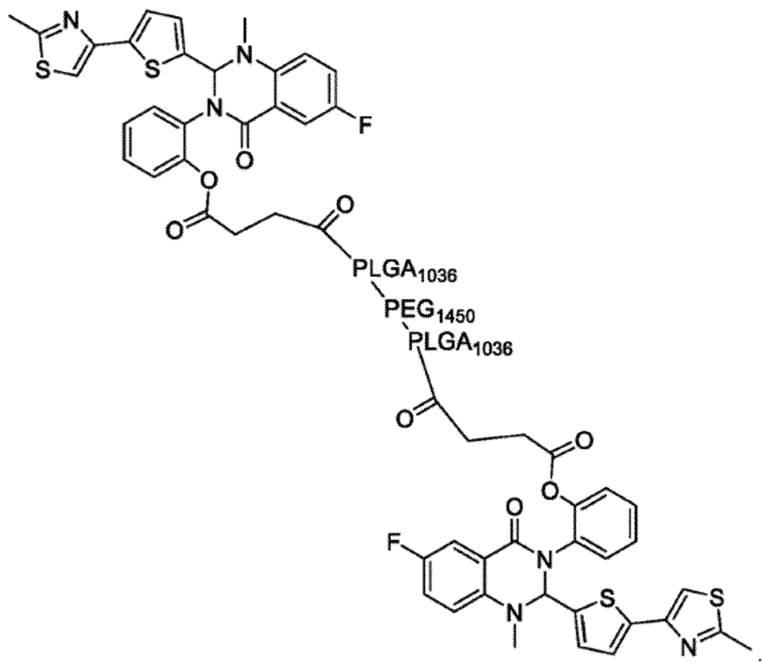

**8.** The compound of general formula (I) as defined in any one of claims 1 to 7, for use in the prevention and/or treatment of disorders induced by toxins with an intracellular mode of action using the retrograde transport selected from ricin, Shiga toxin and Shiga-like toxins (Stxs) produced by *Shigella dysenteriae* (Stx) and *E. coli,* cholera toxin, pertussis toxin, cytotoxin subtilase, and heat-labile enterotoxin, by virus using the retrograde and/or syntaxin 5-dependent transport to infect cells, selected from pox viruses, cytomegalovirus, adenoviruses and adeno-associated viruses, polyomavirus, papillomavirus, filovirus, enterovirus, herpes virus, virus of the genus *Arenavirus,* influenza virus, and pneumovirus, bacteria using the retrograde and/or syntaxin 5-dependent transport to infect cells, chosen from bacteria of the order Chlamydiales or *Symkania,* or by intracellular parasites.

**9.** The compound for its use according to claim 8, **characterized in that** said toxins with an intracellular mode of action are chosen from ricin, Shiga toxin and Shiga-like toxins (Stxs) produced by *Shigella dysenteriae* (Stx) and *E. coli* (Stx1 and Stx2), cholera toxin (Ctx from *Vibrio cholerae* responsible for cholera), pertussis toxin (*Bordetella pertussis* agent of whooping cough), cytotoxin subtilase and heat-labile enterotoxin (*E. coli*).

**10.** The compound for its use according to claim 8, **characterized in that** said viruses are pox viruses, in particular smallpox virus, monkeypox virus, vaccinia virus and leporipoxviruses, in particular myxomatosis virus, cytomegaloviruses, adeno-associated viruses, in particular of serotype 2, polyomaviruses, in particular polyomavirus JC and polyomavirus BK, papillomaviruses, filoviruses, in particular Ebola virus and Marburg virus, enteroviruses, in particular enterovirus 71, herpes virus, in particular Herpes simplex virus type 2, virus of the genus *Arenavirus,* in particular lymphocytic choriomeningitis virus, influenza virus, in particular influenzavirus A, pneumovirus, in particular respiratory syncytial virus.

**11.** A pharmaceutical composition or medicament comprising at least one compound of general formula (I) as defined

in any one of claims 1 to 7 as active ingredient, and a pharmaceutically acceptable vehicle, said pharmaceutical composition or said medicament being suitable in particular for administration by the air, oral, parenteral or topical routes.

Figure 1

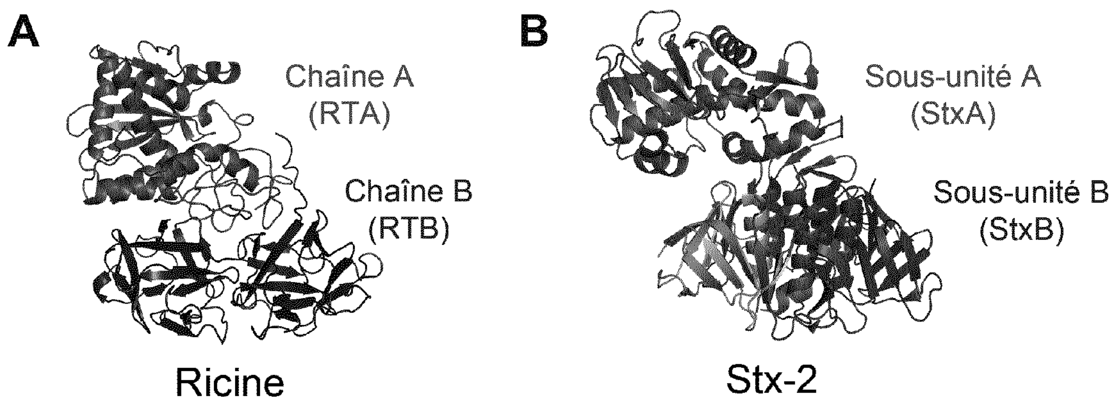


Figure 2

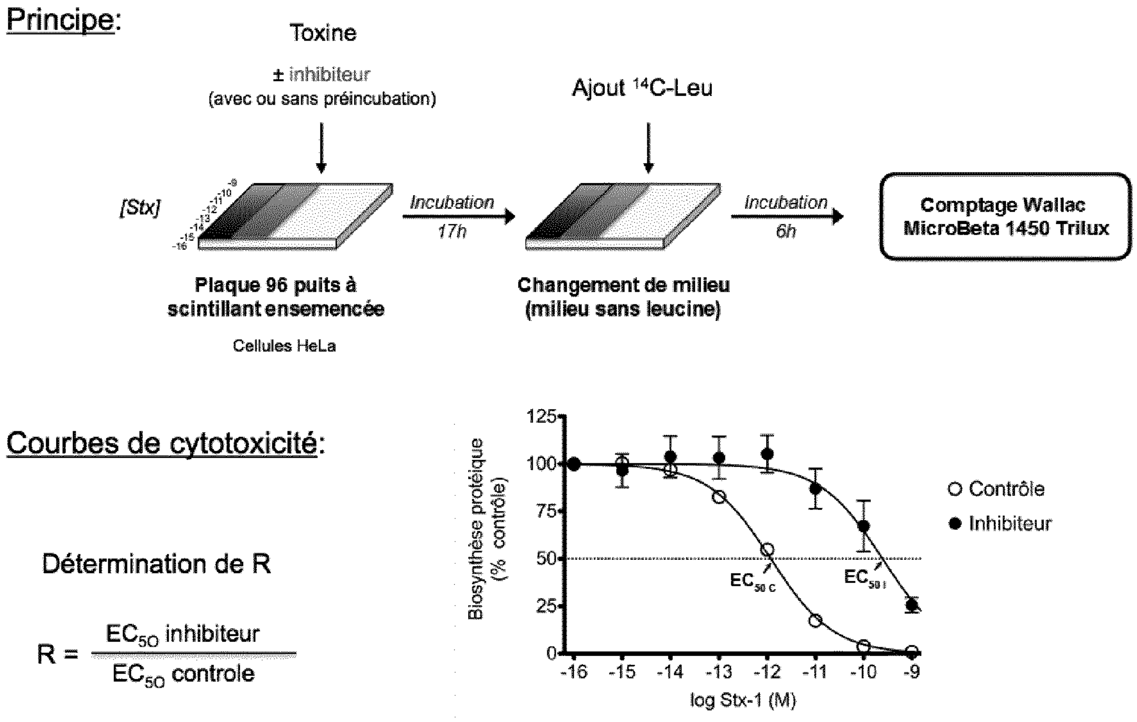

**Figure 3**

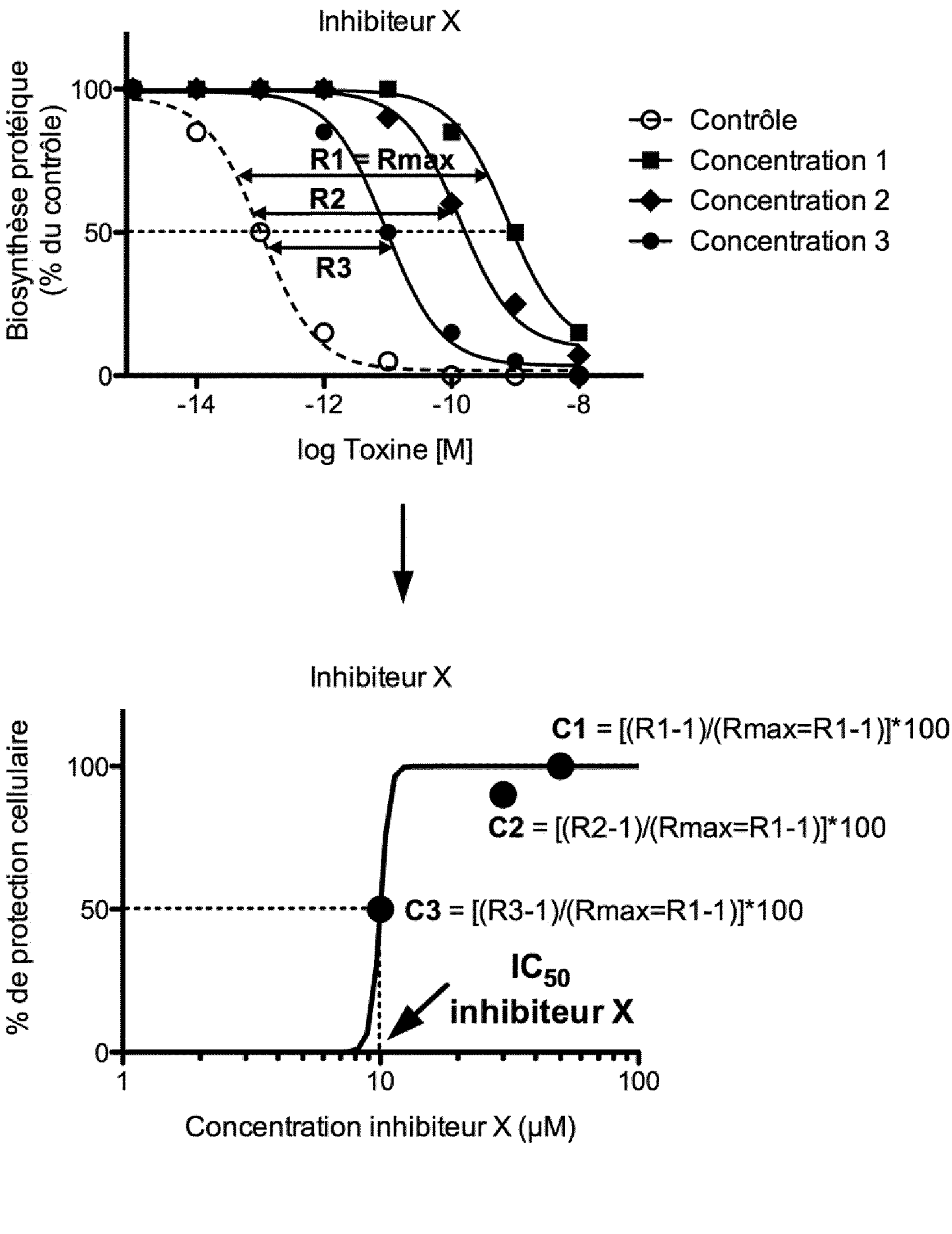
Inhibiteur X
Biosynthèse protéique (% du contrôle)
100
50
0
-14
-12
-10
-8
log Toxine [M]
R1 = Rmax
R2
R3
Contrôle
Concentration 1
Concentration 2
Concentration 3
Inhibiteur X
% de protection cellulaire
100
50
0
1
10
100
Concentration inhibiteur X (µM)
C1 = [(R1-1)/(Rmax=R1-1)]*100
C2 = [(R2-1)/(Rmax=R1-1)]*100
C3 = [(R3-1)/(Rmax=R1-1)]*100
IC50 inhibiteur X

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **JOHANNES, L. et al.** *Cell,* 2008, vol. 135, 1175-87
- **LORD, J.M. et al.** *Biochemical Society Transactions,* 2003, vol. 31, 1260
- **ROMAIN NOEL et al.** N -Methyldihydroquinazolinone Dérivatives of Retro-2 with Enhanced Efficacy against Shiga Toxin. *JOURNAL OF MEDICINAL CHEMISTRY,* 25 Avril 2013, vol. 56, 3404-3413
- **NEETU GUPTA et al.** S )- N -Methyldihydroquinazolinones are the Active Enantiomers of Retro-2 Derived Compounds against Toxins. *ACS MEDICINAL CHEMISTRY LETTERS,* 04 Décembre 2013, ISSN 1948-5875
- **R.C. LAROCK.** Comprehensive Organic Transformations. Wiley-VCH Publishers, 1999
- **T.W. GREENE ; P. G. M. WUTS.** Protective Groups in Organic Chemistry. John Wiley and Sons, 1999
- **J. F. W. MCOMIE.** Protective Groups in Organic Chemistry. Plénum Press, 1973
- **TOWNER et al.** *Virology,* 2005, vol. 332, 20-7
- **DI VEROLI et al.** *Bioinformatics,* 2016, vol. 32 (18), 2866-8